# EUROPEAN PATENT SPECIFICATION

(11) **EP 3 517 967 B1**
(45) Date of publication and mention of the grant of the patent: **23.08.2023**
(21) Application number: 18153664.0
(22) Date of filing: 26.01.2018
(51) Int. Cl.: G01N 33/74, C12N 5/09

(54) **SCREENING METHOD FOR ESTROGENIC AND ANTI-ESTROGENIC ACTIVITY**
SCREENING-VERFAHREN FÜR ÖSTROGENE UND ANTI-ÖSTROGENE AKTIVITÄT
PROCÉDÉ DE CRIBLAGE D'ACTIVITÉ ESTROGÈNIQUE ET ANTI-ESTROGÈNIQUE

(43) Date of publication of application: 31.07.2019
(73) Proprietor: Bundesinstitut Für Risikobewertung, 10589 Berlin (DE)
(72) Inventor: Schönfelder, Gilbert, 12247 Berlin (DE); Oelgeschläger, Michael, 15831 Mahlow (DE); Bischoff, Philip, 10437 Berlin (DE); Kornhuber, Marja, 10629 Berlin (DE); Dunst, Sebastian, 14513 Teltow (DE); Vogl, Silvia, 10587 Berlin (DE)
(74) Representative: Rückerl, Florian

(56) References cited:
- M KORNHUBER ET AL: "Adherens junction reorganization as a novel endpoint for the identification of estrogenic substances", NAUNYN-SCHMIEDEBERG'S ARCHIVES OF PHARMACOLOGY, vol. 390, no. Suppl. 1, 1 March 2017 (2017-03-01), page S7, XP055473039, Berlin, DE ISSN: 1432-1912
- C A HEINRICH ET AL: "Negative regulation of N-cadherin-mediated cell-cell adhesion by the estrogen receptor signaling pathway in rat pituitary GH3 cells.", ENDOCRINE, vol. 10, no. 1, 1 February 1999 (1999-02-01), pages 67-76, XP055472989, US ISSN: 1355-008X
- WANG SI ET AL: "A low-density DNA microchip for the detection of (anti-)estrogenic compounds and their relative potencies", ANALYTICAL BIOCHEMISTRY, ELSEVIER, AMSTERDAM, NL, vol. 435, no. 1, 4 January 2013 (2013-01-04), pages 83-92, XP028979477, ISSN: 0003-2697, DOI: 10.1016/J.AB.2012.12.016
- SI WANG ET AL: "Proliferation assays for estrogenicity testing with high predictive value for theuterotrophic effect", JOURNAL OF STEROID BIOCHEMISTRY AND MOLECULAR BIOLOGY, ELSEVIER SCIENCE LTD., OXFORD, GB, vol. 128, no. 3, 16 November 2011 (2011-11-16), pages 98-106, XP028438323, ISSN: 0960-0760, DOI: 10.1016/J.JSBMB.2011.11.009 [retrieved on 2011-11-25]

## Description

### BACKGROUND OF THE INVENTION

### I. Field of the Invention

The present invention relates to a cell as deposited under accession number DSM ACC3321 and recombinant clones thereof forming discontinuous basolateral adherens junctions in presence of an anti-estrogenic compound. Furthermore, the present invention relates to methods for testing a compound of interest for potential estrogenic or anti-estrogenic activity as well as to methods for testing a cell for its potential to form discontinuous basolateral adherens junctions in presence of an anti-estrogenic compound. The application relates also to uses of a cell capable of forming discontinuous basolateral adherens junctions. The present invention relates furthermore to *ex vivo* methods of analysing the morphology of adherens junctions in tissue of a subject.

### II. Description of Prior Art

Many developmental and general bodily functions in animate beings are determined and regulated through hormones excreted by the glands of the endocrine system into the circulating system to act on distant organs. Hormones that are transported through the bloodstream to their target cells bind to specific receptors localized on the cell surface or in the cytosol, which in turn can induce changes in gene expression and enzymatic activities.

In the last decades there is an increasing concern that chemicals might interact with the endocrine system causing severe adverse effects in humans and wildlife. Generally, there is evidence towards endocrine disruption of the male and female reproductive system in wildlife. For example, the abundance of different reproductive disorders like decreased testes size or baculum length seen in male polar bears could be linked to a bioaccumulation of organohalogen pollutants. Changes in sex-ratios towards more female offspring were observed in non-mammalian vertebrates like roach downstream wastewater effluents that contained estrogenic contaminants. For humans, the incidences for several diseases associated with the endocrine system like obesity, neurobehavioral disorders, endocrine-related cancers or genital malformations have been described to be rising. A prominent example for a link between exposure to an endocrine substance and hormone-related cancer risk is the pharmaceutical diethylstilbestrol (DES), a potent estrogenic substance. During the 1940s to 1970s DES was given to pregnant women in order to reduce the risk of pregnancy complications and to prevent miscarriages. Later it was shown that daughters exposed to diethylstilbestrol *in utero* had a higher risk of developing adenocarcinomas of the vagina at a young age or breast cancer later in life.

Substances that can interact with the endocrine system ("endocrine active substances") can be found nearly ubiquitously. Industrial and pharmaceutical chemicals are often not fully removed from waste water in sewage plants and can leach into the environment. Furthermore, many chemicals are part of everyday use like pesticides, plastic items or chemicals used in personal care products. In addition, there are natural substances with endocrine activity, for example isoflavones like genistein and daidzein that are present in various herbs and plants including soybeans. Thus, the contact with endocrine active chemicals can occur through a variety of different exposure routes like oral (food, water), dermal or by inhalation. However, endocrine active substances have to be distinguished from endocrine disruptors. An endocrine disruptor is defined as an "exogenous substance or mixture that alters function(s) of the endocrine system and consequently causes adverse health effects in an intact organism, or its progeny, or (sub) populations" (IPCS (2002). Global assessment of the state-of-the-science of endocrine disruptors. Geneva, Switzerland). In contrast, endocrine active substances do not necessarily impose a threat to human health but can even have beneficial effects, as for example described for isoflavones.

The European regulations for chemicals (Regulation (E) No 1907/2006), biocides (Regulation (EC) No 528/2012) and plant protection products (Regulation (EC) No 1107/2009) strictly impede or prohibit the marketing of chemicals that are classified as endocrine disruptors in Europe.

Thus, there is a need in the art for methods to reliably determine for new compounds the estrogenic potential as well as the associated risk of endocrine disruption.

There are several types of different *in vivo* assays for the identification of endocrine disruptors that act on the estrogen system. One of them is the uterotrophic assay that has been adopted by the OECD in 2007, meaning that data from this assay has to be accepted by regulatory authorities of all OECD member states. Immature or ovariectomised female rats or mice are treated with the test substance for three successive days, before they are necropsied 24 hours after the last dose. The weight of the uterine is measured and compared to the control group. For the sensitivity of this assay it is essential that the animals do not have a functional hypothalamic-pituitary-gonadal axis and thereby low endogenous estrogen levels. Estrogen agonists will stimulate weight gain in the uterus due to water imbibition which is followed by weight gain due to tissue growth. Therefore, a significant increase of the mean uterine weight of the treated animal group compared to the control group indicates a biological action of the chemical similar to natural estrogens. Another assay is the pubertal female rat assay (OCSPP Guideline 890.1450) that is used by the US EPA Endocrine Disruptor Screening Program (EDSP) as part of tier 1 in the tiered testing strategy for the identification of endocrine disruptors. Starting at the postnatal day 22, the juvenile female rats are treated through an oral gavage daily with two different doses of the substance with the highest concentration being as close as possible at the maximum tolerated dose. The animals are weighed daily and checked for the different aspects of the estrus cycling (vaginal opening, length and regularity). Necropsy is performed at postnatal day 42 and additional endpoints such as thyroid serum concentrations, classic serum chemistry, weight and histology of several organs are recorded. Disadvantages of these two assays are for example the need for high numbers of animal experimentation as well as the long time span.

There are also various *in vitro* and *in chemico* assays to detect compounds that interact with the estrogen signalling pathway. Estrogen receptor binding assays consist of a saturation binding experiment followed by a competitive binding experiment. The saturation binding experiment is usually conducted with 17-β-estradiol. In the competitive binding experiments different concentrations of the test substance compete then with labelled 17-β-estradiol at one fixed concentration for binding to the estrogen receptor and the release of 17-β-estradiol is determined. However, the test neither does supply information on whether the compound acts as an estrogen receptor agonist or antagonist nor if the binding is associated with a corresponding gene expression response. There are also several reporter gene assays available. In these transactivation assays, a functional interaction of the test substance with the estrogen receptor is measured by the quantification of transcriptional activation or repression of the expression of a reporter gene, such as luciferase or lacZ, that is regulated by an artificial estrogen-response element in the promoter region. Finally, an assay that allows the identification of effects on the biosynthesis of steroid hormones has been developed (H295R Steroidogenesis Assay) that, like the receptor binding and transactivation assays, has received regulatory acceptance as an OECD test guideline. Here, H295R cells that synthesize endogenous steroid hormones are exposed for 48 h to seven concentrations of the test chemical. At the end of the exposure period, the culture medium is removed and the concentrations of 17β-estradiol and testosterone is determined in the medium by standard techniques including commercial ELISA kits or liquid chromatography-mass spectrometry (LC-MS).

Disadvantages of the existing *in vitro* assays are that they do not cover all possible mode-of-actions of an estrogenic or anti-estrogenic compound. This is particularly true if only a single method is used, but might also be true for a combination of test methods. In addition, they are in one way or another artificial and, in particular, do not reflect toxicokinetic aspects of absorption, distribution, metabolism and excretion (ADME). Therefore, positive results of *in vitro* methods do not necessarily correlate with a final adverse effect.

The influence of estrogen signals in the development of cancer, in particular breast cancer, is well established. Anti-estrogenic compounds like tamoxifen or fulvestrant are commonly used in the treatment of estrogen-receptor alpha-positive cancer types. The so-called E-Screen has been established for quite some time to quantify estrogen receptormediated proliferation of the breast cancer cell line MCF-7 as a read-out to detect estrogenic as well as anti-estrogenic activities (Soto AM et al. Environ Health Perspect. 1995 Oct; 103 Suppl 7:113-122).

In the field of regulatory toxicology the identification of molecular pathways that mediate toxicity, so-called "adverse outcome pathways" (AOPs), have become more and more important during the last years. This concept is based on the assumption that a comprehensive understanding of the interaction(s) of toxic substances with molecular factors as well as of the subsequent reactions of cells, organs or organ systems that eventually lead to an adverse effect in an organism will allow the identification of effective and highly predictive *in vitro* testing batteries to identify or exclude hazards for human health. Over 150 AOPs have already been developed for various adverse effects. However, with respect to carcinogenesis, the AOPs for endocrine disruptors mainly focus on changes in proliferative capacity, as do *in vivo* screening systems like the uterotrophic assay. The recently described ER Model of the US EPA is combining 18 *in vitro* test systems, including receptor binding and transactivation assays as well as the E-Screen, to identify and characterize endocrine disruptors acting on the estrogen system. The *in vitro* readout is validated against the uterotrophic assay and, thus, is again addressing the regulation of proliferation by estrogenic signals as an adverse effect. However, proliferation is only one of various aspects of cancer development where evading growth suppressors, resisting cell death, inducing angiogenesis, replicative immortality as well as activation of invasion and metastasis play important roles as well. In sum, the *in vivo* as well as *in vitro* assays that are available today focus on sexual differentiation and proliferation and do not address other relevant aspects of carcinogenesis. For now, two-generation or Extended-one-generation studies (OECD test guidelines 416 and 443) are still needed in order to be able to achieve an acceptable level of safety for the evaluation of endocrine disruptors. These assays are not specifically designed for endocrine mode-of-actions. They rather allow a comprehensive analysis of various adverse effects but are very cost and time consuming. They also require a high number of animals per substance and given the immense number of industrial chemicals that are marketed in Europe, these assays are hardly suitable for a stringent program of identification and analysis of endocrine disruptors.

The published article Kornhuber et al. (Naunyn-Schmiedeberg's Archives of Pharmacology; 2017; 390; Suppl. 1: S7) reports about adherens junction reorganization as a novel endpoint for the identification of estrogenic substances. Heinrich et al. (Endocrine;1999; 10(1):67-76) discloses the negative regulation of N-cadherin-mediated cellcell adhesion by the estrogen receptor signalling pathway in rat pituitary GH3 cells. Wang et al. (Analytical Biochemistry; 2013; 435(1): 83-92) established proliferation assays for estrogenicity testing based on the human MCF-7/BOS breast cancer cell line. Furthermore, Wang et al (Journal of Steroid Biochemistry and Molecular Biology; 2011; 128(3):98-106) developed a low-density DNA microchip for the detection of (anti-)estrogenic compounds, and also utilized the MCF-7/BOS breast cancer cell line.

However, there is still a need in the art for new methods to determine the estrogenic or anti-estrogenic potential of a given compound of interest and it was the objective of the inventors to provide new means for doing so, with means overcoming some of the above mentioned disadvantages being particularly preferred.

This problem is solved by the subject-matter as set forth below and in the appended claims.

### SUMMARY OF THE INVENTION

The inventors of the present invention have surprisingly found that a particular MCF-7 (Michigan Cancer Foundation-7) cell clone established in their laboratory exhibits previously undescribed surprising morphological changes at adherens junctions upon contact with anti-estrogenic substances such as fulvestrant. This property is unique to this MCF-7 clone and is for example not found in the parental cell line MCF-7/BOS (Michigan Cancer Foundation-7/BOS; Soto & Sonnenschein, J Steroid Biochem, 1985, DOI: 10.1016/0022-4731(85)90265-1, Soto et al., Environ Health Perspect. 1995, PMCID: PMC1518887) or the MCF-7/ACC115 cell line (Leibniz-Institut DSMZ - Deutsche Sammlung von Mikroorganismen und Zellkulturen GmbH, Braunschweig, Germany) that both originate from a pleural effusion taken from a human patient with metastatic breast adenocarcinoma (Soule, H. D., Vazguez, J., Long, A., Albert, S., Brennan, M. (1973). A human cell line from a pleural effusion derived from a breast carcinoma. Journal of the National Cancer Institute 51 ( 5 ): 1409-1416). Due to the link between anti-estrogenic and estrogenic effects, this finding can be exploited in several ways. The morphological changes at the adherens junctions can be used to determine the anti-estrogenic properties of a given compound of interest (i.e. the morphological change can be induced in said cell) as well as for determining the estrogenic properties of a compound of interest (i.e. the morphological change induced by an anti-estrogenic compound is prevented, reduced or reversed). In addition, further cells and cell lines may be screened in view of the invention for the ability to form discontinuous basolateral adherens junctions in presence of an anti-estrogenic compound.

Therefore, the present invention relates in a first aspect to a cell as deposited under DSM ACC3321 or a cell deriving from the cell as deposited under DSM ACC3321 and forming discontinuous basolateral adherens junctions in presence of an anti-estrogenic compound, wherein the cell deriving from the cell as deposited under DSM ACC3321 is a recombinant clone of said cell.

In a second aspect, the present invention relates to an *in vitro* method for testing a compound of interest for potential estrogenic activity (and/or level thereof), the method comprising the following steps:
a) growing a cell in presence of an anti-estrogenic compound and in in presence of the compound of interest, wherein growing the cell in presence of the anti-estrogenic compound may precede and/or occur in parallel to growing the cell in presence of the compound of interest, and
b) determining the adherens junctions morphology of the cell,
wherein the cell is a cell responding to the exposure to an anti-estrogenic compound by formation of discontinuous basolateral adherens junctions, and wherein prevention, reduction or reversion, respectively, of a discontinuous basolateral adherens junctions phenotype is indicative of estrogenic activity of the compound of interest.

In a third aspect, the present invention relates to an *in vitro* method for testing a compound of interest for potential anti-estrogenic activity (and/or level thereof), the method comprising the following steps:
a) growing a cell in presence of the compound of interest, and
b) determining the adherens junction morphology of the cell,
wherein the cell is a cell responding to the exposure to an anti-estrogenic compound by formation of discontinuous basolateral adherens junctions, and wherein a formation of discontinuous basolateral adherens junctions is indicative of anti-estrogenic activity of the compound of interest.

Furthermore, the present invention relates in a fourth aspect to an *in vitro* method for testing a cell for its potential to form discontinuous basolateral adherens junctions in presence of an anti-estrogenic compound, the method comprising the following steps:
a) growing the cell in presence of an anti-estrogenic compound, and
b) determining the adherens junction morphology of the cell.

In a fifth aspect, the present invention relates to *ex vivo* methods assessing the basolateral adherens junction morphology of cells in a tissue sample of a subject, the method comprising determining in the tissue sample of the subject the fraction of cells exhibiting discontinuous basolateral adherens junctions and/or determining in the tissue sample of the subject the fraction of cells exhibiting continuous basolateral adherens junctions. Such method may take the form of a method for assessing the metastatic potential of a tumour in a subject, wherein a reduced fraction of cells exhibiting discontinuous basolateral adherens junctions compared to a control and/or an increased fraction of cells exhibiting continuous basolateral adherens junctions compared to a control indicates an elevated metastatic potential of the tumour and thus an increased risk of metastasis. The method of the fifth aspect may also be a method for assessing the efficacy of tumour therapy in a subject suffering from cancer, wherein an essentially similar or increased fraction of cells exhibiting discontinuous basolateral adherens junctions and/or an essentially similar or reduced fraction of cells exhibiting continuous basolateral adherens junctions compared to prior treatment indicates an efficacious therapy.

The present invention relates in a sixth aspect to the use of a cell forming discontinuous basolateral adherens junctions in presence of an anti-estrogenic compound for:
a) testing a compound of interest for potential anti-estrogenic activity and/or level of anti-estrogenic activity, and/or for
b) testing a compound of interest for potential estrogenic activity and/or level of estrogenic activity.

### DESCRIPTION OF ILLUSTRATIVE EMBODIMENTS

### I. Definitions

As used herein, the term "compound of interest" refers to an individual compound as well as to more complex compositions comprising more than one chemical entity.

The use of the word "a" or "an", when used herein, may mean "one," but it is also consistent with the meaning of "one or more," "at least one," and "one or more than one." In particular, if herein reference is made to using "a" compound, said embodiment does also encompass situations in which mixtures of compounds (i.e. compositions) are used. For example, if herein reference is made to "growing a cell in presence of an anti-estrogenic compound", then growing the cells in presence of a single anti-estrogenic compound such as fulvestrant but also in presence of a mixture of anti-estrogenic compounds (e.g. fulvestrant and tamoxifen) is contemplated.

Furthermore, as a person skilled in the art will readily understand, "growing a cell" is not meant to refer to a single, individual cell, but to a cell culture comprising a multitude of said cells. Presence of additional other cell types is not excluded, but not preferred. Moreover, a person skilled in the art will understand that growing a cell is typically accomplished under standard conditions for cell culture. Unless otherwise stated, growing a cell does therefore not encompass growing a cell under conditions where all compounds exerting estrogenic activity have artificially been fully depleted.

### II. Cell line MCF7/vBOS

As mentioned previously, the present invention is based on the surprising discovery, that a particular MCF-7 (Michigan Cancer Foundation-7) cell clone established in the laboratory of the inventors exhibits previously undescribed surprising morphological changes at adherens junctions upon contact with anti-estrogenic substances such as fulvestrant. Said cell line, MCF7/vBOS (Michigan Cancer Foundation-7/variantBOS), has been deposited at the Leibniz-Institut DSMZ-Deutsche Sammlung von Mikroorganismen und Zellkulturen GmbH (Braunschweig, Germany) and is listed under accession number DSM ACC3321. It has not been made available to the public otherwise. Said cell line is derived from a subclone of the parental cell line MCF-7/BOS (Michigan Cancer Foundation-7/BOS; Soto & Sonnenschein, J Steroid Biochem, 1985, DOI: 10.1016/0022-4731(85)90265-1, Soto et al., Environ Health Perspect. 1995, PMCID: PMC1518887) that originates from a pleural effusion taken from a human patient with metastatic breast adenocarcinoma. MCF7/vBOS is characterized by a pronounced reorganization of cell adhesion complexes upon treatment with estrogen antagonists, like fulvestrant or tamoxifen. Such cell, or a recombinant clone deriving from MCF7/vBOS as deposited under DSM ACC3321 can be used for determining anti-estrogenic and/or estrogenic activity of compounds or composition, e.g. by carrying out the methods of the present invention.

### III. Methods for testing potential estrogenic activity

The present invention provides a method for testing a compound of interest for potential estrogenic activity (and/or level thereof), the method comprising the following steps:
a) growing a cell in presence of an anti-estrogenic compound and in presence of the compound of interest, wherein growing the cell in presence of the anti-estrogenic compound may precede and/or occur in parallel to growing the cell in presence of the compound of interest, and
b) determining the adherens junctions morphology of the cell,
wherein the cell is a cell responding to the exposure to an anti-estrogenic compound by formation of discontinuous basolateral adherens junctions, and wherein prevention, reduction or reversion, respectively, of a discontinuous basolateral adherens junctions phenotype is indicative of estrogenic activity of the compound of interest.

The compound of interest may be any type of compound (or composition) for which it is desirous to test its estrogenic properties. Preferably, the compound of interest is not 17-β-estradiol (E2), in particular not if the cell is a cell as deposited under DSM ACC3321.

In principle the cell to be used with the inventive method of testing a compound of interest for potential estrogenic activity may be any cell capable of forming discontinuous basolateral adherens junctions in presence of an anti-estrogenic compound. Adherens junctions with comparable structures, including E-Cadherin, can be found throughout the animal kingdom. Similarly, nuclear receptors that can respond to estrogens and estrogen-like proteins have been identified not only in vertebrate but also in invertebrate species. Preferably the cell derives from a vertebrate. Most preferably (in particular for regulatory purposes), the cell is a mammalian cell, such as an isolated human cell. The cell may be immortalized. For example, the cell may be an adenocarcinoma cell. Particularly useful are breast cancer (adenocarcinoma) cells. Preferably, the cell is estrogen receptor alpha positive. Most preferably, a cell according to the present invention, such as the cell deposited under accession number DSM ACC3321, is used in the inventive method for testing a compound of interest for potential estrogenic activity (and/or level thereof).

The anti-estrogenic compound to be used with the inventive method of testing a compound of interest for potential estrogenic activity (and/or level thereof) may be any compound with known anti-estrogenic activity on the cell to be used in the method. A person skilled in the art will be readily aware of a number of such compounds and conventional tests for ascertaining anti-estrogenic properties in the cell to be used. Standard operating procedures for various *in vitro* test methods accepted by the OECD, e.g. OECD test guidelines 455, 456 or 493, for estrogen receptor binding, transactivation or steroidogenesis assays, are available and suitable reference chemicals can be found in these test guidelines as well as in the supplementary performance standards (OECD Series on Testing and Assessment, No 173, 174). Particularly useful anti-estrogenic compounds, in particular for human cells (such as a cell of the invention) are fulvestrant, tamoxifen, ZK 164015, and MPP dihydrochloride, with fulvestrant being the most preferred anti-estrogenic compound. While the use of an individual anti-estrogenic compound is preferred, the inventors also contemplate the use of mixtures of anti-estrogenic compounds.

Growing the cells in presence of the anti-estrogenic compounds will typically be achieved by simply adding the anti-estrogenic compound to the cell culture medium or by using fresh cell culture medium containing the anti-estrogenic compound. A possible culture medium, without being limited to said particular type of medium, is Dulbecco's modified Eagle's medium (e.g. DMEM, Biochrom, FG 0415) containing 10% Fetal Bovine Serum (e.g. FBS, Biochrom, S 0615, LOT 1353W), with low endogenous estrogen levels (below 10⁻¹⁰ M 17-β-Estradiol) additionally supplemented with 100 µg/ml streptomycin / 100 U/ml penicillin (e.g. Biochrom, A 2212), in particular in cases where the cell is a cell as deposited under accession number DSM ACC3321. Using a medium with endogenous estrogen levels as low as possible (theoretically even without any estrogenic substance) is preferred, but the invention is not limited thereto. If media with normal or high endogenous estrogen levels are used, then the levels of anti-estrogenic compound should preferably be adjusted accordingly. The inventors also contemplate a further specific alternative for this facet of the invention, where the cell is (apart from the potential estrogenic activity of the compound of interest) grown under conditions devoid of any other estrogen (e.g. by artificial depletion of any such compounds from the medium and utilization of inert reagents and materials). In such scenario, i.e. absence of any estrogenic activity (aside of the potential activity of the compound of interest), discontinuous basolateral adherens junctions phenotype can manifest, even in absence of an anti-estrogenic compound (which is thus dispensable in said scenario).

A cell according to the present invention will - typically after exposure to an anti-estrogen and sufficient cell culture period - manifest a discontinuous basolateral adherens junctions phenotype. Without being bound to any theory, the inventors speculate that the underlying principle of the method of testing a compound of interest for potential estrogenic activity (and/or level thereof) is that an estrogenic compound would "antagonize" the phenotypic effect induced by an anti-estrogen in cells according to the present invention.

The interplay between anti-estrogen and (potential) estrogen can be implemented in different ways depending on whether growing the cell in presence of the anti-estrogenic compound precedes and/or occurs in parallel to growing the cell in presence of the compound of interest. In other words, it depends on the time point at which the compound of interest is allowed to antagonize the effect of the anti-estrogen. Among others, the following approaches are for example feasible:
a) the cell is exposed to the anti-estrogen and compound of interest simultaneously,
b) the cell is first exposed to the anti-estrogen and the compound of interest is added subsequently additionally to the medium, but before discontinuous basolateral adherens junctions are formed,
c) the cell is first exposed to the anti-estrogen and the compound of interest is added subsequently additionally to the medium after discontinuous basolateral adherens junctions have formed,
d) the cell is first exposed to the anti-estrogen and the compound of interest replaces subsequently the anti-estrogen in the medium, but before discontinuous basolateral adherens junctions are formed,
e) the cell is first exposed to the anti-estrogen and the compound of interest replaces subsequently the anti-estrogen in the medium after discontinuous basolateral adherens junctions have formed.

In those cases where the compound of interest is added before discontinuous basolateral adherens junctions have formed, a compound of interest (provided it exhibits estrogenic properties) might completely prevent formation of said discontinuous basolateral adherens junctions (i.e. the effect of the anti-estrogen is negated) or would at least reduce the extent of formation. In those instances where the cell is contacted with the compound of interest only after discontinuous basolateral adherens junctions have formed, an estrogenic compound may reduce or even reverse the phenotype. For example, the method for testing potential estrogenic activity may involve that the cells are *ab initio* prevented from forming discontinuous basolateral adherens junctions in presence of an anti-estrogenic compound such as fulvestrant. For this purpose, the cell will usually be grown in a medium, containing at the same time said anti-estrogenic compound and the compound of interest, for a time usually sufficient to allow formation of the discontinuous basolateral adherens junctions in control cells that are exposed to the anti-estrogenic compound alone.

The time usually required for formation of discontinuous basolateral adherens junctions will depend on the estrogen receptor binding affinity and concentration of the anti-estrogen used on the one hand and the cell used on the other hand, but a person skilled in the art will be readily capable of determining the appropriate time spans and concentration for a given anti-estrogen and cell. For example, if the cell is a cell as deposited under accession number DSM ACC3321, suitable concentration ranges and culture times for manifestation of the discontinuous basolateral adherens junctions phenotype in presence of fulvestrant, tamoxifen, ZK 164015 and MPP dihydrochloride, without being limited thereto, are:

**Table 1**

| Anti-estrogenic compound | CAS No | Concentration (M) | Culture time (h) |
|---|---|---|---|
| Fulvestrant / ICI 182,780 | 129453-61-8 | 10⁻⁹ - 10⁻⁷ | 24 - 96 |
| Tamoxifen | 10540-29-1 | 10⁻⁶ - 10⁻⁵ | 30 - 168 |
| ZK 164015 | 177583-70-9 | 10⁻⁷ - 10⁻⁵ | 24 - 96 |
| MPP dihydrochloride | 911295-24-4 | 10⁻⁶ - 10⁻⁵ | 30 - 168 |

It has to be noted that the culture time indicated above in table 1 is the time span typically required for formation of discontinuous basolateral adherens junctions in presence of an anti-estrogenic compound. However, this does not necessarily imply that the anti-estrogenic compound must be inevitably present throughout said culture period (although this is the preferred embodiment). For example, the inventors have found that exposure to fulvestrant for only 2 hours does likewise lead to manifestation of the discontinuous basolateral adherens junctions phenotype after, e.g., 48 hours in cells cultured at that time in media w/o fulvestrant. Possible culture periods in which the anti-estrogen is present are for example, in particular for fulvestrant, at least 2, 3, 4, 5, 6, 7, 8, 9, 10, 12, 14, 16, 18, 20, 22, 24, 30, 36, 42 or at least 48 hours. The same applies for the compound of interest, i.e. it need not necessarily be present throughout the entire culture period, although this is the preferred embodiment. Possible culture periods regarding presence of the compound of interest are for example, in particular if fulvestrant is used as opposing anti-estrogen, at least 2, 3, 4, 5, 6, 7, 8, 9, 10, 12, 14, 16, 18, 20, 22, 24, 30, 36, 42 or at least 48 hours. In a preferred embodiment the length of the culture period in which the anti-estrogen is present is identical to the length of the culture period in which the compound of interest is present (e.g., both compounds are present in parallel).

If the cell is not cultured in a medium containing the anti-estrogen and the compound of interest in parallel, then the gap between the (first) period of growing the cell in presence of an anti-estrogenic compound and the (second) period of growing the cell in presence of the compound of interest is preferably less than 48 hours, preferably less than 24 hours, more preferably less than 12 hours, more preferably less than 6 hours, more preferably less 5, 4, 3, 2, or less than 1 hour. In said gap the cell is preferably cultured in the same media as before and/or afterwards, merely w/o anti-estrogen and compound of interest.

In order to test a compound of interest for estrogenic activity, it is preferred to apply the compound of interest together with a fixed concentration of an anti-estrogen (preferably fulvestrant) that will induce discontinuous basolateral adherens junctions in a reliable and robust manner (10⁻⁸ M for fulvestrant). To derive a quantitative dose-response relationship, preferably at least five and even more preferably at least 11 different concentrations of the compound of interest are tested with an upper concentration limit defined by the limit of solubility of the compound of interest or the induction of significant cell death. As a starting point the compound should be applied in a concentration range from 10⁻¹⁰ to 10⁻⁴ M if these concentrations do not affect cell viability and are below the limit of solubility of the test compound. Preferably, the method will be carried in multiplicates. It is also possible to set up this method as high-throughput screening, wherein a multitude of compounds of interests and/or concentrations of anti-estrogen and/or concentrations of the compound of interest are tested in parallel.

As mentioned previously, in one embodiment the compound of interest may be added to the cell culture after discontinuous basolateral adherens junctions have formed in the cell (e.g. a cell according to the present invention). Here, the cells will first be grown in presence of the anti-estrogen to induce formation of discontinuous basolateral adherens junctions. Evidently, in these embodiments where the compound of interest is added only after formation of discontinuous basolateral adherens junctions, the method may optionally comprise prior to the step of growing the cell in presence of the compound of interest a step in which formation of discontinuous basolateral adherens junctions is determined/verified. After discontinuous basolateral adherens junctions have stably formed (typically within 28-48 hours for fulvestrant, see table 1 above), the compound of interest is applied for a time sufficient for an estrogenic compound to reduce or reverse the fraction of cells displaying discontinuous basolateral adherens junctions. The discontinuous basolateral adherens junctions are stable without adding additional anti-estrogens for at least another 72 hours and, thus, the reduction or reversion of cells displaying discontinuous basolateral adherens junctions during the following, e.g., 24 to 48 hours after addition of the compound of interest can be taken as a measure for estrogenic activity, in presence and even in absence of the anti-estrogen. Therefore, typical time spans for growing the cell in presence of the compound of interest are in this scenario between 24 to 48 hours, without being limited thereto.

After growing the cell in presence of an anti-estrogenic compound and the compound of interest, the method for testing a compound of interest for potential estrogenic activity (and/or level thereof) according to the invention requires that the adherens junction morphology of the cell is determined. For example, after 20 to 200 hours, preferably after 24 to 168 hours (more preferably 24 to 48 hours for fulvestrant, see table 1 above) the cells are checked for the development of discontinuous basolateral adherens junctions. The fraction of cells displaying continuous basolateral adherens junctions may for example be taken as a measure for estrogenic activity of the compound of interest. Here, a substance with high estrogenic activity would efficiently prevent the formation of discontinuous basolateral adherens junctions resulting in a large fraction of cells displaying continuous basolateral adherens junctions and a small fraction of cells displaying discontinuous basolateral adherens junctions, respectively. In turn, a small fraction of cells displaying continuous basolateral adherens junctions or a large fraction of cells displaying discontinuous basolateral adherens junctions, respectively, indicates low or no estrogenic activity of the compound of interest as it could only partially or not prevent the morphological change. A compound is considered estrogenic only if it can interfere with the formation of discontinuous basolateral adherens junctions as usually induced by the anti-estrogenic substance but without negatively affecting cell viability in parallel. Typically, determining the adherens junction morphology of the cell will be done immediately after growing the cell in presence of the anti-estrogenic compound and the compound of interest for the indicated time, but it is also conceivable that the cells are for example fixed or frozen and the adherens junction morphology is assessed at a later stage.

Suitable controls for the method for testing potential estrogenic activity are cells neither exposed to the anti-estrogen nor the compound of interest, cells exposed to the anti-estrogen but not to the compound of interest, cells exposed to the compound of interest but not to the anti-estrogen and/or cells exposed to the anti-estrogen and a known estrogen.

A suitable approach for assessing the adherens junction morphology of the cell is to do so by optical means, e.g., by way of fluorescence microscopy, in particular by way of confocal fluorescence microscopy. For example, it is possible to stain E-cadherin in the cell via conventional immunostaining techniques and analyse the distribution of E-cadherin at the adherens junctions using confocal, immunofluorescence microscopy. Further possible markers are other membrane bound or membrane associated proteins, in particular those which are components of the adherens junctions. Further markers, without being limited thereto, are for example β-Catenin, α-Catenin, p120-Catenin, and the cortical actin cytoskeleton. Alternatively, the cells might be stained with dyes revealing the morphology of the cell (such as membrane specific or cytosolic dyes). A morphological change of the appearance of the cellular membrane, also visible in pronounced spacing between the cells with cytosolic dyes, is directly correlated with the reorganisation of the adherens junctions. It can also be detected in a quantitative manner using confocal, immunofluorescence microscopy and an appropriate software. Examples for such dyes are cell-permeant nonfluorescent ester of an amine-reactive fluorescent molecule, CellTrace^{®} dye or LysoTracker^{®} (both ThermoFisher Scientific). Another alternative is labelling of F-actin, e.g. via fluorescently (or otherwise) labelled antibodies or conjugated probes. Any other method of determining morphological changes of a cell, in particular of the membrane, will also be useful in assessing the adherens junction morphology of the cell and a person skilled in the art will be readily capable of devising other possibilities.

Once the adherens junction morphology has been determined it will be apparent whether said morphology is rather comparable to the morphology of a negative control (e.g. cells exposed to the anti-estrogen only) and/or a positive control (e.g. cells exposed to the anti-estrogen and a known estrogen). Said controls are preferably carried out in parallel, but it is also conceivable to conduct such control experiments in advance or subsequent to the method of the present invention. Such evaluation process can also be automated, as will be described further down below.

In a preferred embodiment of the method for testing a compound of interest for potential estrogenic activity according to the invention, the cell is a cell as deposited under DSM ACC3321 and the anti-estrogenic compound is fulvestrant.

### IV. Methods for testing potential anti-estrogenic activity

In a further aspect, the present invention relates to a method for testing a compound of interest for potential anti-estrogenic activity (and/or level thereof), the method comprising the following steps:
a) growing a cell in presence of the compound of interest, and
b) determining the adherens junction morphology of the cell,
wherein the cell is a cell responding to the exposure to an anti-estrogenic compound by formation of discontinuous basolateral adherens junctions, and wherein formation of discontinuous basolateral adherens junctions is indicative of anti-estrogenic activity.

The compound of interest may be any type of compound (or composition) for which it is desirous to test its anti-estrogenic properties. Preferably, the compound of interest is neither fulvestrant, tamoxifen, ZK 164015 nor MPP dihydrochloride, in particular not if the cell is a cell as deposited under DSM ACC3321, as for these compounds the anti-estrogenic potential is already known and has already been tested by the inventors.

The cell to be used in the method of the third aspect of the invention is the same as the one described above for the method of the second aspect of the invention. Preferably the cell derives from a vertebrate. Most preferably, the cell is a mammalian cell, such as an isolated human cell. The cell may be immortalized. For example, the cell may be an adenocarcinoma cell. Particularly useful are breast cancer (adenocarcinoma) cells. Preferably, the cell is estrogen receptor alpha positive. Most preferably, a cell according to the present invention, such as the cell deposited under accession number DSM ACC3321, is used in the inventive method for testing a compound of interest for potential anti-estrogenic activity (and/or level thereof).

Growing the cells in presence of the compound of interest will typically be achieved in the method of the third aspect of the invention by simply adding the compound of interest to the cell culture medium or by using fresh cell culture medium containing said compound of interest. A possible culture medium, without being limited to said particular type of medium, is Dulbecco's modified Eagle's medium (e.g. DMEM, Biochrom, FG 0415) containing 10% low-estrogen Fetal Bovine Serum (e.g. FBS, Biochrom, S 0615, LOT 1353W), additionally supplemented with 100 µg/ml streptomycin / 100 U/ml penicillin (e.g. Biochrom, A 2212), in particular in cases where the cell is a cell as deposited under accession number DSM ACC3321. Using a medium with low endogenous estrogen levels (theoretically even without any estrogenic substance, see above) is recommendable, but the method is not limited thereto.

Typically, the method for testing potential anti-estrogenic activity requires that the cell is grown in presence of the compound of interest. For this purpose the cell will usually be grown in a medium containing said compound of interest for a time sufficient to allow formation of discontinuous basolateral adherens junctions. The time required for formation of discontinuous basolateral adherens junctions will depend on the estrogen receptor binding affinity of the compound of interest and concentration used. Reasonable concentrations to be tested may for example be: 10⁻⁴ M, 10⁻⁵ M, 10⁻⁶ M, 10⁻⁷ M, 10⁻⁸ M, 10⁻⁹ M, 10⁻¹⁰ M, 10⁻¹¹ M or 10⁻¹² M, depending on the limit of solubility of the test compound and concentration-dependent effects on cell viability. Incubation times may range for example from about 6 h to about 200h, about 6h to about 168 h, about 12h to about 168h, about 24h to about 168h, about 12 h to about 200h, about 12 h to about 168 h, about 24h to about 200h, about 24h to about 168h, about 24h to about 96h, about 24h to about 72h, about 24h to about 48h, about 24h to 72h, about 48h to 72h, etc. and any combinations thereof. Preferably, the method will be carried in multiplicates using at least five, more preferably at least 10 different concentrations of the compound of interest. It is also possible to set up this method as high-throughput screening, wherein a multitude of compounds of interests and/or concentrations of the compound of interest are tested in parallel.

After growing the cell in presence of the compound of interest, the cells are checked for the development of discontinuous basolateral adherens junctions. The fraction of cells displaying discontinuous basolateral adherens junctions may for example be taken as a measure for anti-estrogenic activity of the compound of interest. Here, a large fraction of cells displaying discontinuous basolateral adherens junctions (or a small fraction of cells displaying continuous basolateral adherens junctions) indicates high anti-estrogenic activity of the compound of interest. In turn, a substance with low anti-estrogenic activity would not be capable of inducing significant formation of discontinuous basolateral adherens junctions resulting in a small fraction of cells displaying such discontinuous basolateral adherens junctions (or a high fraction of cells displaying continuous basolateral adherens junctions, respectively). A compound is considered anti-estrogenic if the compound by itself can induce discontinuous basolateral adherens junctions within, e.g., 168 hours of exposure at a concentration that does not interfere with cell viability.

Suitable controls for the method for testing potential anti-estrogenic activity are for example corresponding cells not exposed to the compound of interest, cells exposed to compound known to be inactive in this assay, or corresponding cells exposed to a known anti-estrogen, such as fulvestrant.

The inventive method of testing a compound of interest for potential anti-estrogenic activity encompasses a step in which the adherens junctions morphology of the cell is determined. As it will not be known in advance how long it takes for formation of the discontinuous basolateral adherens junctions in presence of the compound of interest, this step can be performed at different times, e.g., after 24, 48, 72, 96, 120, 144, 168 and/or 192 hours. Typically, determining the adherens junction morphology of the cell will be done immediately after the period of growing the cell in presence of the anti-estrogenic compound and the compound of interest, but it is also conceivable that the cells are for example fixed or frozen and the adherens junction morphology is assessed at a later stage.

Suitable approaches for assessing the adherens junction morphology of the cell have already been discussed above for the method according to the second aspect of the invention and are applicable for the method according to the third aspect as well. A preferred approach is the assessment via optical means, e.g., by way of confocal microscopy. For example, it is possible to stain E-cadherin in the cell via conventional immunostaining techniques and analyse the distribution of E-cadherin at the adherens junctions using immunofluorescence microscopy and a confocal microscope with appropriate software.

Once the adherens junctions morphology has been determined it will be apparent whether said morphology is rather comparable to the morphology of a positive control (e.g. cells exposed to a known anti-estrogen such as fulvestrant) or a negative control (e.g. corresponding cells not exposed to the compound of interest or a compound known to be inactive in this assay). Said controls may be carried out in parallel, but it is also possible to conduct such control experiments in advance or subsequent to the method of the present invention. Such evaluation process can also be automated, as will be described further down below.

In a preferred embodiment of the method for testing a compound of interest for potential anti-estrogenic activity according to the invention, the cell is a cell as deposited under DSM ACC3321.

### V. Methods for testing a cell for its potential to form discontinuous basolateral adherens junctions

In a fourth aspect the present invention relates to an *in vitro* method for testing a cell of interest for its potential to form discontinuous basolateral adherens junctions in presence of an anti-estrogenic compound, the method comprising the following steps:
a) optionally growing the cell of interest in presence of an anti-estrogenic compound, and
b) determining the adherens junction morphology of the cell of interest.

While the cell line as deposited under DSM ACC3321 is currently the preferred cell line for carrying out the inventive methods of testing compound of interest for potential estrogenic or anti-estrogenic activity, there may be other cell types and cell lines, which also form discontinuous basolateral adherens junctions upon contact with an anti-estrogenic compound. A suitable test for identifying such candidate cells will typically require that the candidate cell is exposed to an anti-estrogenic compound such as fulvestrant, tamoxifen, ZK 164015, and MPP dihydrochloride, with fulvestrant being the most preferred anti-estrogenic compound. Another possible but less preferred assay would be an assay carried out under conditions absolutely free of any estrogenic activity, e.g. by depleting all estrogenic compounds from the medium and/using inert materials and culture devices etc.. In such 100% estrogen-free environment (which is usually hardly attainable under normal conditions), a cell according the present invention may already manifest a discontinuous basolateral adherens junctions phenotype even in absence of any specific anti-estrogen.

The cell of interest may be any type of cell for which it is desirous to test its potential to form discontinuous basolateral adherens junctions. In principle the cell to be tested with the inventive method of the fourth aspect of the invention may be any cell capable of forming basolateral adherens junctions. Preferably the candidate cell derives from a vertebrate. Most preferably, the cell is a mammalian cell, such as an isolated human cell. The cell may be immortalized. For example, the cell may be an adenocarcinoma cell. Particularly interesting are breast cancer (adenocarcinoma) cells. In some embodiments, the cell is a cell isolated previously from a subject. Preferably, the cell is estrogen receptor alpha positive. Preferably, the cell of interest is not a cell as deposited under DSM ACC3321, as for this clone the potential to form discontinuous adherens junctions upon contact with an anti-estrogen is already known and has already been tested by the inventors. However, a cell deriving from a cell as deposited under DSM ACC3321 is a preferred cell of interest for the fourth aspect of the invention.

The anti-estrogenic compound to be used with the method of the fourth aspect of the invention may be any compound with known anti-estrogenic activity and may be the same as discussed above in the context of the second and/or third aspect of the invention. Particularly useful anti-estrogenic compounds are fulvestrant, tamoxifen, ZK 164015, and MPP dihydrochloride, with fulvestrant being the most preferred anti-estrogenic compound to be used in the context of this aspect of the invention. While the use of an individual anti-estrogenic compound is preferred, the inventors also contemplate the use of mixtures of anti-estrogenic compounds.

The cell of interest will typically be grown in the type of media usually required for maintenance of said cell, preferably with low estrogen levels. Moreover, the method will be carried out preferably in multiplicates using different concentrations of the anti-estrogenic compounds. This will allow rapid assessment of the potential of the candidate cell to form discontinuous basolateral adherens junctions. For example, and without being limited thereto, if the anti-estrogen is fulvestrant, the following concentrations could be tested: 10⁻¹¹ M, 10⁻¹⁰ M, 10⁻⁹ M, 10⁻⁸ M, 10⁻⁷ M, 10⁻⁶ M, 10⁻⁵ M. Preferred concentrations for fulvestrant, tamoxifen, ZK 164015, and MPP dihydrochloride are presented in table 1 above. It is also possible to set up this method as high-throughput screening, wherein a multitude of candidate cells is tested in parallel.

Preferably, the cell of interest is incubated with the anti-estrogenic compound for at least 2 to at least 200 hours. Preferred time spans for fulvestrant, tamoxifen, ZK 164015, and MPP dihydrochloride are presented in table 1 above. In particular, if the anti-estrogenic compound is fulvestrant, then the cell of interest is incubated with the anti-estrogenic compound preferably for at least 2 to 24 hours, preferably for at least 24 to 48 hours, most preferably for 48 hours.

The adherens junction morphology is determined in this method in analogous manner as described above for the inventive methods of the second and third aspect of the invention. Particularly preferred embodiments will utilize optical means and immunofluorescence, such as E-cadherin staining and confocal microscopy. A suitable positive control and reference cell is a cell as deposited under accession number DSM ACC3321, possible negative control and reference cells could be either MCF-7/BOS cells (Prof. Ana Soto, Tufts University School of Medicine, Boston, MA, USA) or MCF-7/ACC115 (DSMZ). Once the adherens junction morphology has been determined it will be apparent whether the morphology of the cell of interest (after exposure to an anti-estrogen and appropriate culture time usually sufficient for manifestation of the phenotype in other responsive cells) is rather comparable to the morphology of a negative control or a positive control.

### VI. Further methods involving determining the basolateral aherens junction morphology

In a further (fifth) aspect the present invention relates to a method (preferably an *ex vivo* method) for assessing the basolateral adherens junctions morphology of cells in a tissue sample of a subject, the method comprising determining in the tissue sample of the subject the fraction of cells exhibiting discontinuous basolateral adherens junctions and/or determining in the tissue sample of the subject the fraction of cells exhibiting continuous basolateral adherens junctions.

The inventors of the present invention discovered, that the diverging organization of basolateral adherens junctions can also occur in the *in vivo* situation. Therefore, imaging of the organization of basolateral adherens junctions does also have its application in the field of, e.g., analysis of tissue samples obtained from a living being. Preferably, the tissue sample is a section of a tumour or a section of a tissue suspected of being a tumour. The tumour may be in some embodiments an estrogen receptor alpha positive tumour and/or a breast tumour.

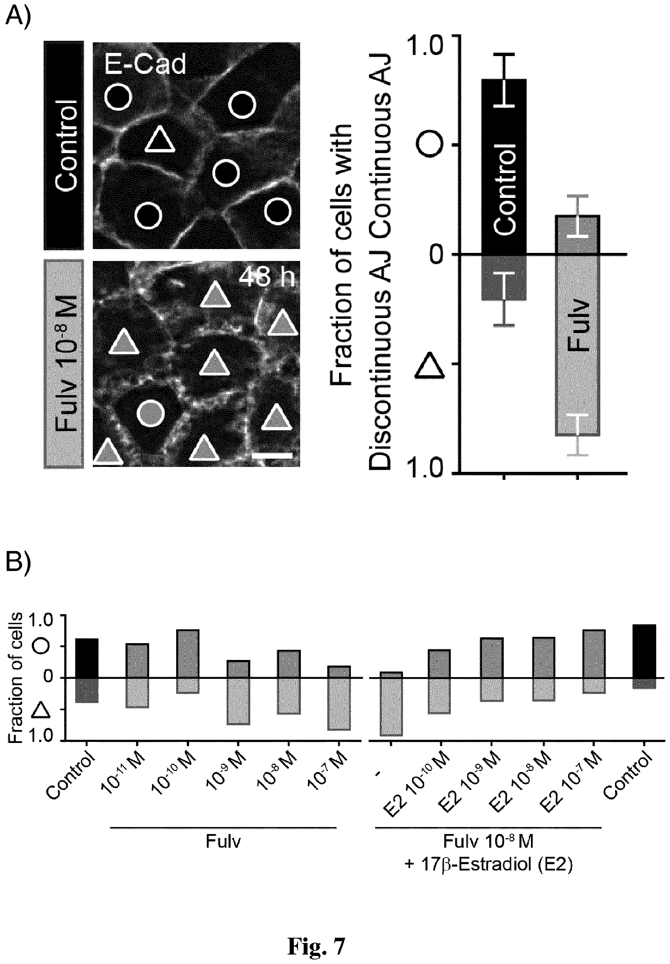

The adherens junction morphology is determined in this fifth aspect in analogous manner as described above for the inventive methods of the second, third or fourth aspect of the invention. Particularly preferred embodiments will utilize optical means and immunofluorescence, such as E-cadherin staining, confocal microscopy and/or optical sectioning fluorescence microscopy. The assessment of adherens junction morphology may also occur in an automated manner, as set out for instance in Fig. 7 and example 7.

Additionally, the inventors have discovered that in estrogen receptor alpha positive samples a correlation between a discontinuous basolateral adherens junction phenotype and cytoplasmic localization of estrogen receptor alpha, and vice versa a correlation between a continuous basolateral adherens junction phenotype and nuclear localization of estrogen receptor alpha can be observed. A cytoplasmic localization of estrogen receptor alpha corresponds to a low level of estrogen receptor alpha signalling while nuclear estrogen receptor alpha indicates a high signalling level. Therefore, in embodiments where the tissue sample is estrogen receptor alpha positive (e.g., a section of an estrogen receptor alpha positive tumour or a section of a tissue suspected of being an estrogen receptor alpha positive tumour or the like), the method may additionally comprise the step of determining in the tissue sample of the subject the fraction of cells exhibiting essentially cytoplasmatic localisation of estrogen receptor alpha and/or the step of determining in the tissue sample of the subject the fraction of cells exhibiting essentially nuclear localisation of estrogen receptor alpha. Analysis of both parameters (adherens junction morphology and subcellular localisation of estrogen receptor alpha) will yield a more detailed picture of the status of the tissue under scrutiny. Analysis of the subcellular localisation of estrogen receptor alpha may occur prior to, concomitant with or after analysis of the basolateral adherens junction morphology.

The subject is preferably a human subject. The subject may be male or female, in particular female. The subject may suffer from cancer, in particular from an estrogen receptor alpha positive cancer. The subject may suffer in particular from breast cancer, endometrial cancer, ovarian cancer, lung cancer, thyroid cancer, prostate cancer and/or pancreatic cancer, in particular from estrogen receptor alpha positive forms thereof. In many embodiments the cancer will be breast cancer, in particular an estrogen receptor alpha positive breast cancer.

The inventors of the present invention also discovered that a continuous adherens junction phenotype correlates with reduced cell rigidity and increased cell motility, and vice versa. Reduced cell rigidity and increased cell motility in turn reflect an increased metastatic potential.

Thus, the present invention also relates in a specific embodiment of this aspect of the invention to a method (preferably carried out *ex vivo*) for assessing the metastatic potential of a tumour in a subject, the method comprising determining in a tissue sample of the tumour the fraction of cells exhibiting discontinuous basolateral adherens junctions and/or determining in the tissue sample of the tumour the fraction of cells exhibiting continuous basolateral adherens junctions, wherein a reduced fraction of cells exhibiting discontinuous basolateral adherens junctions compared to a control and/or an increased fraction of cells exhibiting continuous basolateral adherens junctions compared to a control indicates an elevated metastatic potential of the tumour and thus an increased risk of metastasis. The tumour is preferably estrogen receptor positive and/or is a breast tumour.

The control may be healthy, non-tumour tissue of the same subject. For example, and without being limited thereto, tissue sections of a tumour frequently also contain associated non-tumour tissue, which can serve as control. Results from tissues from other sites of the subject, or results from tissue of one or more healthy subjects may also serve as control.

As before, if the tumour is estrogen receptor positive, then the inventive method for assessing the metastatic potential may also comprise the step of determining in the tissue sample of the subject the fraction of cells exhibiting essentially cytoplasmatic localisation of estrogen receptor alpha and/or the step of determining in the tissue sample of the subject the fraction of cells exhibiting essentially nuclear localisation of estrogen receptor alpha.

As before, the subject is preferably a human subject. The subject may be male or female, in particular female. The subject may suffer from cancer, in particular from an estrogen receptor alpha positive cancer. The subject may suffer in particular from breast cancer, endometrial cancer, ovarian cancer, lung cancer, thyroid cancer, prostate cancer and/or pancreatic cancer, in particular from estrogen receptor alpha positive forms thereof. In many embodiments the cancer will be breast cancer, in particular an estrogen receptor alpha positive breast cancer.

The aforementioned correlation of basolateral adherens junction morphology with cell rigidity and motility also allows monitoring of the efficacy of typical anti-cancer treatments. Therefore, the present invention also relates to a method (in particular an ex vivo method) for assessing the efficacy of tumour therapy in a subject suffering from cancer, the method comprising determining in a tissue sample of the tumour the fraction of cells exhibiting discontinuous basolateral adherens junctions and/or determining in the tissue sample of the tumour the fraction of cells exhibiting continuous basolateral adherens junctions, wherein an essentially similar or increased fraction of cells exhibiting discontinuous basolateral adherens junctions and/or an essentially similar or reduced fraction of cells exhibiting continuous basolateral adherens junctions compared to prior treatment indicates an efficacious therapy. In this respect an essentially similar fraction of cells exhibiting discontinuous or continuous basolateral adherens junctions means that the treatment was capable of preventing further disease progression, while an increased fraction of cells exhibiting discontinuous basolateral adherens junctions (or a decreased fraction of cells exhibiting continuous basolateral adherens junctions) compared to prior treatment indicates that the therapy shifted the basolateral adherens junctions phenotype towards a status less prone for metastasis. Assessment of adherens junction morphology, the cancer type and the subject may be done as defined above. The tumour therapy may for example be a breast cancer therapy, e.g. with selective estrogen receptor modulators (SERMs), such as tamoxifen, toremifene, droloxifene, idoxifene, raloxifene, arzoxifene and EM-800, or selective estrogen receptor down-regulators, such as fulvestrant, SR 16234 and ZK 191703. The method may also be used to adapt the type of cancer therapy to treatment efficacy. For example, if tamoxifen therapy brings about unsatisfactory results in this test, a shift towards the more efficient (in terms of binding to estrogen receptor alpha) fulvestrant may be contemplated.

### VIII. Uses

The present invention relates in a sixth aspect to the use of a cell forming discontinuous basolateral adherens junctions in presence of an anti-estrogenic compound for a) testing a compound of interest for potential anti-estrogenic activity and/or level of anti-estrogenic activity, and/or for b) testing a compound of interest for potential estrogenic activity and/or level of estrogenic activity.

The cell may be any cell as discussed above for the other aspects of the invention, in particular of the first, second, third or fourth aspect. Particularly preferred are
mammalian cells. Most preferred is a cell as deposited under DSM ACC3321 or a cell deriving from such cell. The test for potential estrogenic activity or for potential anti-estrogenic activity may for example be a method as set forth above in the second and third aspect of the invention, respectively.

The present invention also relates to the use of means to detect discontinuous and/or continuous basolateral adherens junctions, for assessing the metastatic potential of an estrogen receptor alpha positive tumour (in particular a breast tumour), and/or for assessing the efficacy of tumour therapy in a subject. In particular such means will be compounds or agents facilitating detection of the morphology of the adherens junctions. Examples for means to detect discontinuous and/or continuous basolateral adherens junctions are for example (e.g., labelled) antibodies directed against membrane bound or membrane associated proteins, in particular those which target components of the adherens junctions such as antibodies against E-cadherin, β-Catenin, α-Catenin, p120-Catenin, and the cortical actin cytoskeleton. Preferably, the antibody is an anti-E-cadherin antibody.

### BRIEF DESCRIPTION OF THE FIGURES

In the following a brief description of the appended figures will be given. The figures are intended to illustrate the present invention in more detail. However, they are not intended to limit the scope of the invention to these specific examples only.

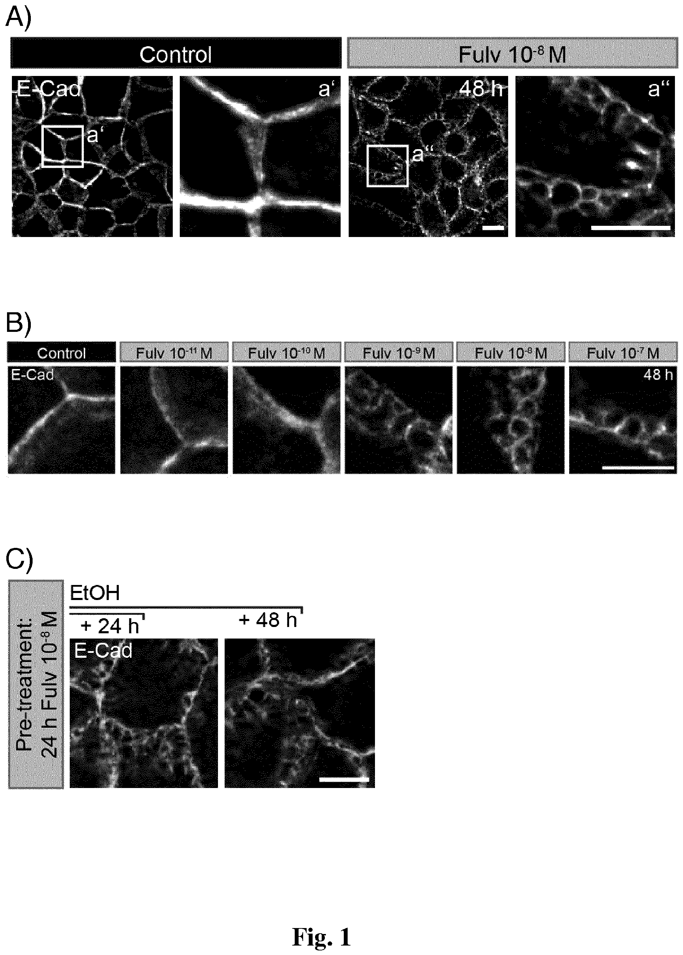

Fig. 1 illustrates by means of E-Cadherin staining the effect of fulvestrant on adherens junction morphology of MCF7/vBOS cells. A) Immunofluorescence images showing increased intercellular spacing and formation of discontinuous adherens junctions demarcated by immunofluorescence staining for E-Cadherin upon fulvestrant treatment for 48 hours (right panel) compared to the solvent control (left panel). White boxes indicate enlarged cell membrane areas shown in a'-a". Scale bars, 10 µm. B) Immunofluorescent staining of MCF7/vBOS cells treated with solvent control or increasing concentrations (10⁻¹¹ to 10⁻⁷ M) of fulvestrant for 48 hours and stained for E-Cadherin demonstrate the appearance of discontinuous adherens junctions upon treatment with a minimal concentration of 10⁻⁹ M fulvestrant for 48 hours. Scale bars, 10 µm. C) Immunofluorescence staining for E-Cadherin showing adherens junction organization after fulvestrant treatment for 24 hours and subsequent cultivation for additional 24 or 48 hours in fulvestrant-free medium containing only the solvent ethanol revealed that discontinuous adherens junctions are stable for at least 48, independent of the presence of fulvestrant. Scale bars, 10 µm.

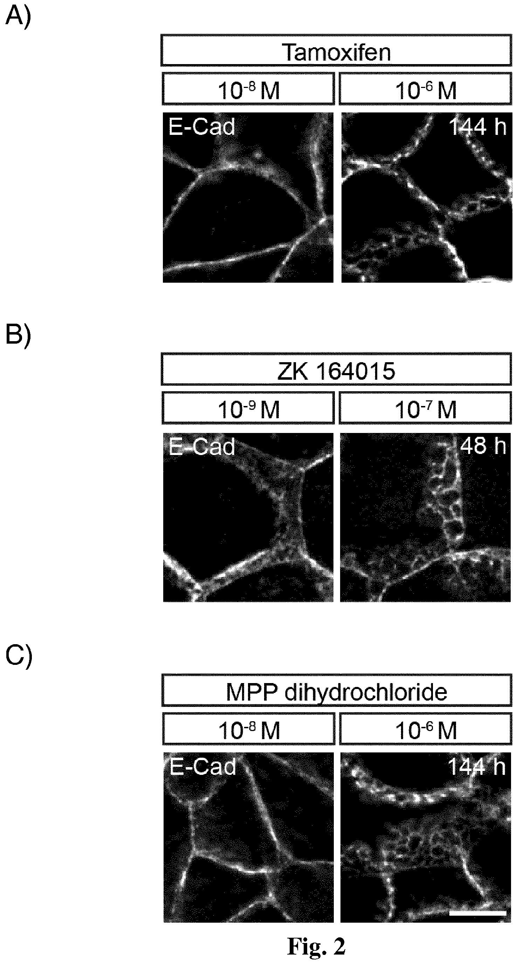

Fig. 2 illustrates that the effect attained with fulvestrant in MCF7/vBOS cells can also be attained with other anti-estrogens such as A) tamoxifen, B) ZK 164015, and C) MPP dihydrochloride. MCF7/vBOS cells were incubated with 10⁻⁸ and 10⁻⁶ M tamoxifen for 144 hours (A), 10⁻⁹ and 10⁻⁷ M ZK164015 for 48 hours (B) or 10⁻⁸ and 10⁻⁶ MPP hydrochloride for 144 hours (C) and immunofluorescence staining for E-Cadherin performed. Discontinuous adherens junctions became apparent with all three anti-estrogens although at different concentrations and after different incubation times. From these data one can also conclude that fulvestrant is more active in this assay than tamoxifen, ZK164015 or MPP. Scale bar, 10 µm.

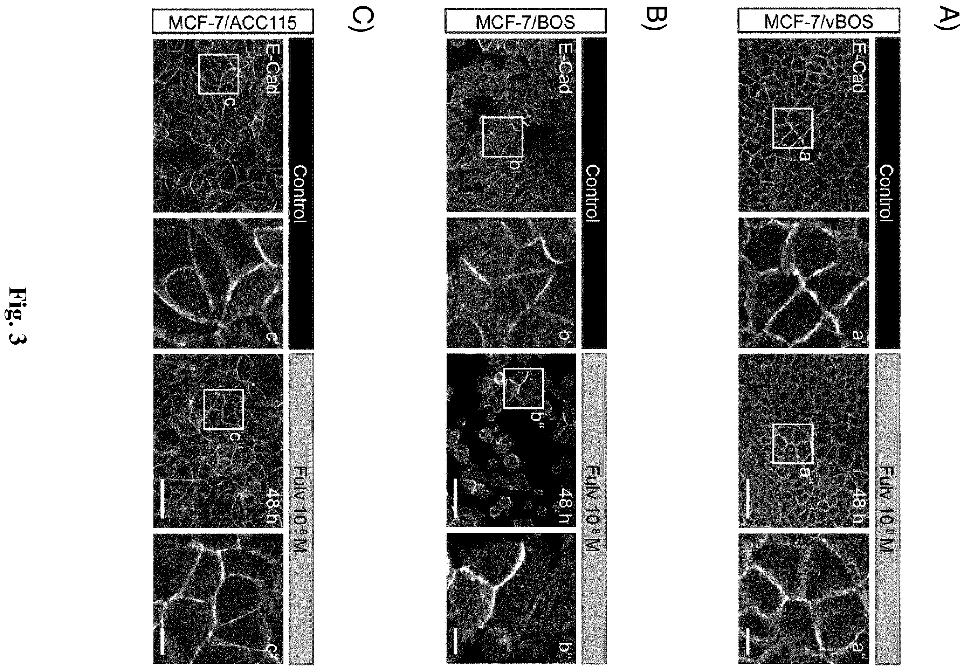

Fig. 3 illustrates by means of E-Cadherin staining that the MCF7/vBOS cell line exhibits unique properties. The figure shows that the MCF7/vBOS cell line (A) exhibits in absence of the anti-estrogenic compound fulvestrant a normal (continuous) basolateral adherens junction morphology (left panel), while said morphology changes in presence of 10⁻⁸ M fulvestrant for 48 hours (right panel). In contrast, the parental MCF-7 subclone MCF-7/BOS (B) as well as the MCF-7/ACC115 cell line (C) do show the same continuous adherens junctions morphology in absence (left panels) and presence of fulvestrant (right panels). White boxes indicate enlarged cell membrane areas shown in a'-c' and a"-c". Scale bars, 10 µm.

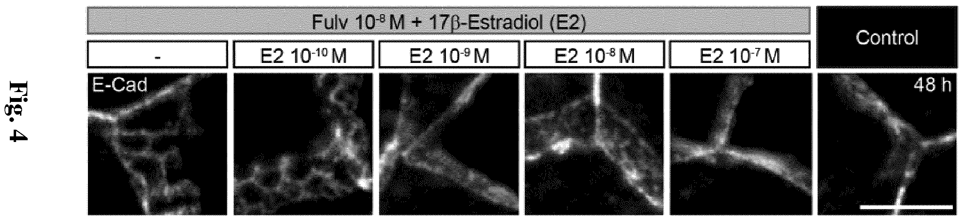

Fig. 4 illustrates the prevention of the formation of discontinuous adherens junctions, if an estrogen, here 17β-estradiol (E2), is additionally present in the cell culture medium along with fulvestrant. Staining of MCF7/vBOS cells for E-Cadherin by immunofluorescence demonstrates that the formation of discontinuous adherens junctions by treatment with 10⁻⁸ M fulvestrant (left picture) can be prevented by co-incubation with increasing concentrations (10⁻¹⁰ to 10⁻⁷ M) of 17β-estradiol (E2). Already co-treatment with 10⁻⁹ M E2 suffices to preserve continuous adherens junctions that are indistinguishable to the one observed in untreated control cell (right picture). Scale bar, 10 µm.

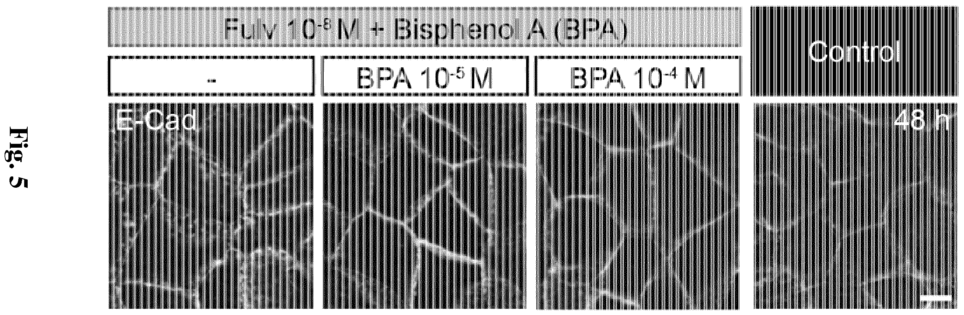

Fig. 5 illustrates again the prevention of the formation of discontinuous adherens junctions, if an estrogenic compound, here bisphenol A, is additionally present in the cell culture medium along with fulvestrant. Immunostaining for E-cadherin on cells treated with 10⁻⁸ M fulvestrant and 10⁻⁵ or 10⁻⁴ M bisphenol A, respectively. Scale bar, 10 µm.

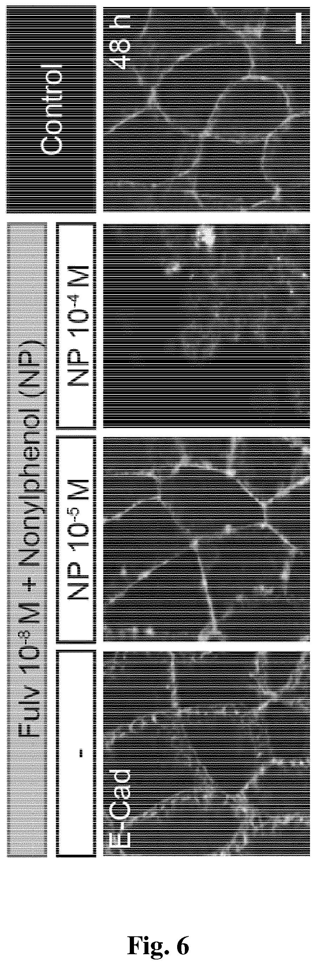

Fig. 6 illustrates again the prevention of the formation of discontinuous adherens junctions, if an estrogenic compound, here nonylphenol, is additionally present in the cell culture medium along with fulvestrant. Immunostaining for E-cadherin on cells treated with 10⁻⁸ M fulvestrant and 10⁻⁵ nonylphenol. Scale bar, 10 µm.
Fig. 7 illustrates the results of an automated quantification of morphology changes at adherens junctions. A) CellProfiler/CellProfiler-Analyst-based quantification of cells displaying continuous and discontinuous AJs. Bars represent the fraction of all cells classified as `Continuous AJ' (circles) and `Discontinuous AJ' (triangles) upon fulvestrant treatment for 48 hours (grey bars) compared to the solvent control (black bars). Biological replicates, n=3. Bars, mean +/- s.d.. Representative pictures of the analysed cells are shown in the left panel that also indicates cells that were classified as `Continuous AJ' (circles) or as `Discontinuous AJ' (triangles) in control or fulvestrant treated cells. B) CellProfiler/CellProfiler-Analyst-based quantification of the fraction of cells displaying continuous AJs (circle) and discontinuous AJs (triangle) (see figure 7 a) upon ERα signalling inhibition (fulvestrant titration, left panel, see figure 1B), and restoration (fulvestrant at 10⁻⁸ M in combination with 17β-estradiol titration; right panel, see figure 4) for 48 hours (grey bars) compared to the solvent control (black bars). Bars, mean of three replicate images.

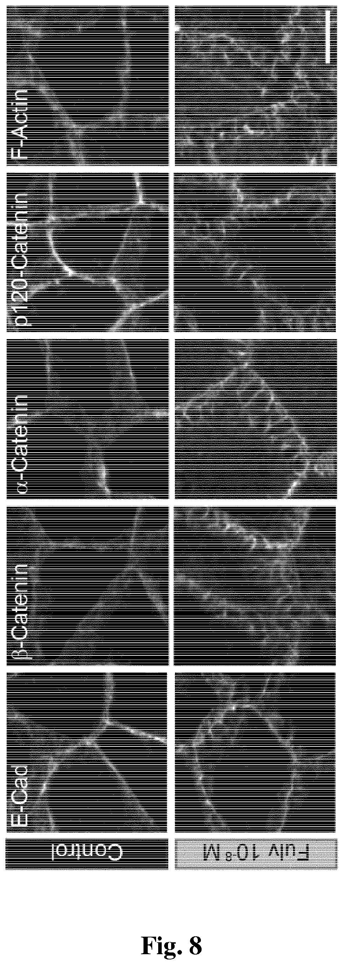

Fig. 8 shows immunofluorescence images illustrating organization of adherens junctions including E-cadherin (E-Cad) and the cytoplasmic adaptor proteins α-Catenin, β-Catenin and p120-Catenin that connect E-Cad to the underlying actomyosin network (F-Actin staining) upon fulvestrant treatment for 48 hours compared to the solvent control. Scale bar, 10 µm.

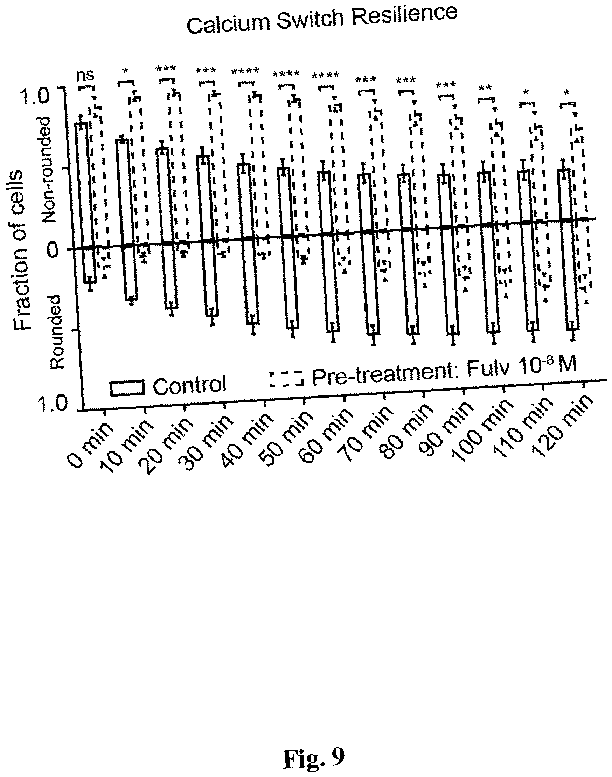

Fig. 9 provides a CellProfiler/CellProfiler-Analyst-based quantification (analogous to Fig. 7) showing the rate of cell rounding over the course of 120 minutes in MCF7/vBOS cells pre-treated with fulvestrant for 48 hours (dotted bars) compared to solvent control cells (black bars). Indicated time points depict time after application of EGTA-containing medium (EGTA, 8×10⁻³ M) to disrupt adherens junctions. Biological replicates, n=3. Error bars, mean +/- s.e.m.. Statistical testing, one-way ANOVA with Sidak's correction for multiple comparisons; ^{∗}, p<0.05; ^{∗∗}, p<0.01; ^{∗∗∗}, p<0.005; ^{∗∗∗∗}, p<0.001. Scale bar, 50 µm.

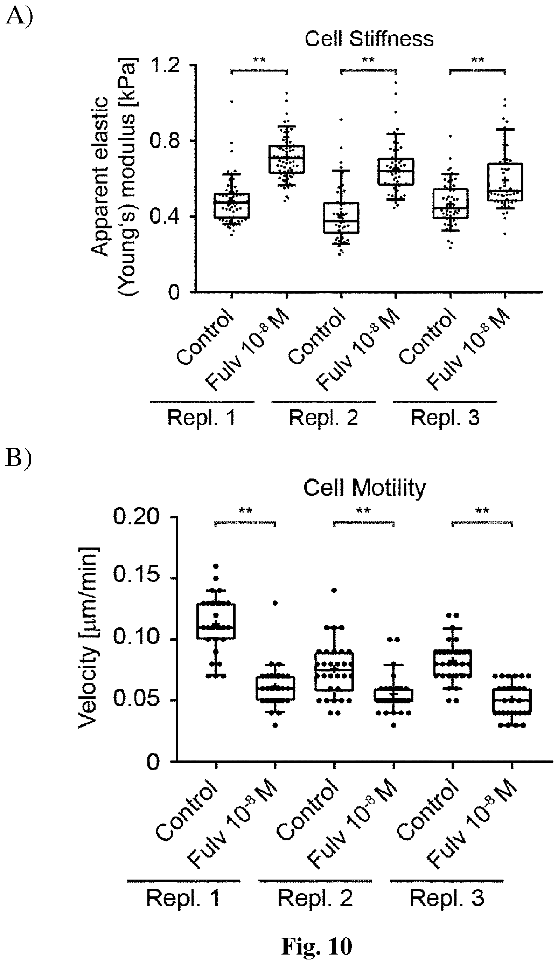

Fig. 10 illustrates that inhibition of ERα signaling increases tissue stability by modulating cell mechanics and cell motility. (A) Quantification of cell stiffness (Apparent elastic (Young's) modulus) by atomic force microscopy (AFM) indentation measurements on cells treated with fulvestrant for 48 hours (E_{Repl.1} = 0.7141 ± 0.1193 kPa; E_{Repl.2} = 0.651 ± 0.1407 kPa; E_{Repl.3} = 0.5938 ± 0.1564 kPa; mean ± s.d.) compared to solvent control cells (, E_{Repl.1} = 0.4841 ± 0.1194 kPa; E_{Repl.2} = 0.4088 ± 0.1513 kPa; E_{Repl.3} = 0.4660 ± 0.1189 kPa). Biological replicates, n=3; 50-60 cells per condition. Boxes, 25th, 50th (median), and 75th percentiles. Whiskers, 10th and 90th percentiles. Cross, mean for each group. Statistical testing, Mann-Whitney tests; ^{∗∗}, p values < 0.01. (B) Quantification of cell motility (velocity) by manual tracking of cells treated with fulvestrant for 48 hours (V_{Repl.1} = 0.0613 ± 0.0176 µm/min; V_{Repl.2} = 0.0553 ± 0.0157 µm/min; V_{Repl.3} = 0.051 ± 0.014 µm/min; mean ± s.d.) compared to solvent control cells (V_{Repl.1} = 0.113 ± 0.0237 µm/min; V_{Repl.2} = 0.0757 ± 0.023 µm/min; V_{Repl.3} = 0.0827 ± 0.0174 µm/min). Biological replicates, n=3; 3 fields with 10 cells each per replicate and condition were imaged every 10 minutes over the course of 16 h. Boxes, 25th, 50th (median), and 75th percentiles. Whiskers, 10th and 90th percentiles. Cross, mean for each group. Statistical testing, Mann-Whitney tests; ^{∗∗}, p values < 0.01.

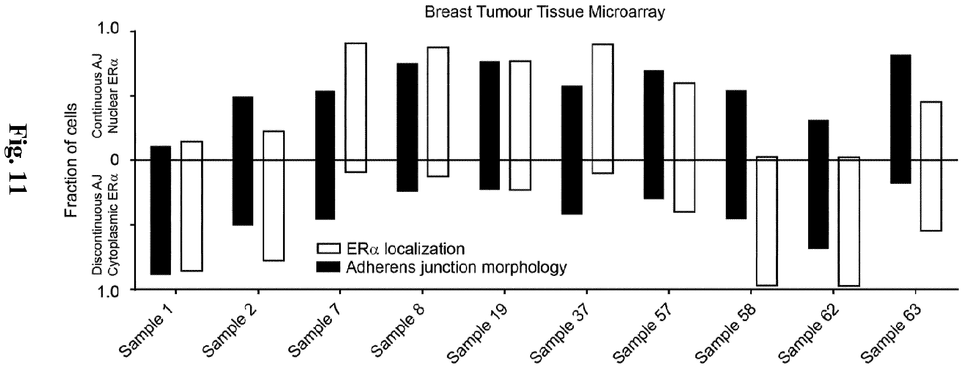

Fig. 11 illustrates that human breast cancer sections can differ in terms of adherens junction organization and that ERα signalling activity correlates with adherens junction organization. The figure shows CellProfiler/CellProfiler-Analyst-based quantification (analogous to Fig. 7) of the fraction of cells displaying continuous or discontinuous adherens junctions (black bars) and nuclear or cytoplasmic ERα localization (white bars) in breast cancer tissue sections of 10 out of 54 sample images that comprised a greater number of detected cells (>60) than used for supervised training of the image analysis pipeline. Each sample represents an individual patient except of samples 1 and 2, which originate from the same patient, and samples 7 and 8, which originate from a further patient. Bars, fraction of cells per image.

### EXAMPLES

In the following, specific examples illustrating various embodiments and aspects of the invention are presented.

### Example 1: Cell line MCF7/vBOS

MCF7/vBOS (Michigan Cancer Foundation-7/variantBOS) cells have evolved from the original human breast adenocarcinoma MCF-7/BOS cell line by natural selection under laboratory conditions. Cell cultures from a cryopreserved master cell bank were routinely maintained in cell culture flasks (TPP) at 37°C with 5% CO2 in low estrogen serum medium (Dulbecco's modified Eagle's medium (DMEM, Biochrom), 10% FBS (Biochrom, S 0615, LOT 1353W), 100 µg/ml streptomycin / 100 U/ml penicillin (Biochrom)). Cells were subcultured at 70-80% confluency over a maximum of 8-10 passages to minimize passage number-related effects, and regularly tested for bacterial contamination using a mycoplasma test service (GATC Biotech AG).

The cell line has been deposited at the Leibniz Institut DSMZ-Deutsche Sammlung von Mikroorganismen und Zellkulturen GmbH (DSZM) under accession number DSM ACC3321.

### Example 2: ERα antagonist fulvestrant triggers formation of discontinuous adherens junctions

MCF7/vBOS cells were grown on glass coverslips in multi-well tissue culture plates (TPP) for 24 h and then treated for at least 48 h with 0.1% EtOH (solvent control) and different concentrations (10⁻¹¹ M, 10⁻¹⁰ M, 10⁻⁹ M, 10⁻⁸ M, 10⁻⁷ M) of the ERα antagonist fulvestrant (Sigma-Aldrich, CAS-No. 129453-61-8). During experiments, medium was exchanged on a daily basis. All experiments were performed at 37°C with 5% CO₂ in reduced-serum medium (phenol red-free DMEM (Gibco), 5% FBS (Biochrom, S 0615, LOT1353 W), 1% glutamine, 100 µg/ml streptomycin / 100 U/ml penicillin (Biochrom)).

After treatment with fulvestrant the adherens junction morphology of the cells was analysed by means of immunofluorescence microscopy. Briefly, cells were fixed with 3.7% formaldehyde in PBS for 15 min, permeabilized with 0.2% Triton X-100 in PBS for 30 min, and blocked with 5% FBS in PBS for 60 min (all steps at room temperature). Incubation with primary and secondary antibodies in PBS containing 1.5% BSA was carried out over night at 4°C and for 1 h at room temperature, respectively. The following antibodies/dyes were used: anti-E-Cadherin (H-108, Santa Cruz); DAPI (Roche); secondary antibody conjugated to Cy3 (alternatives are, e.g.: Cy2, Alexa Fluor 488/555/647; all Invitrogen). The samples were then mounted in Dako fluorescence mounting medium (Dako) or VectaShield (Vector Labs). All images were acquired with an Axio Observer.Z1 widefield microscope equipped with an Apotome.2 device for optical sectioning using structured illumination (Zeiss). After 48 hours the fraction of cells displaying discontinuous adherens junctions was determined by CellProfiler/CellProfer-analyst software (see example 7).

The result of this experiment is shown in figure 1 and 7. Fulvestrant triggers the formation of discontinuous adherence junctions between adjacent MCF7/vBOS cells throughout the epithelial monolayer within two days after treatment. Upon formation, discontinuous cell contacts are even preserved for at least two days without further fulvestrant addition. The discontinuity of cell contacts is restricted to the level of the basolateral adherence junctions marked by E-Cadherin staining.

### Example 3: Other ERα antagonists likewise trigger formation of discontinuous adherens junctions

In view of the results obtained with MCF7/vBOS cells upon exposure to fulvestrant the inventors speculated that a similar phenotype might be obtainable with other ERα antagonist as well. To test this hypothesis, the inventors used the ERα antagonists tamoxifen (Sigma-Aldrich, CAS-No. 10540-29-1), MPP dihydrochloride (Sigma-Aldrich, CAS-No. 911295-24-4), and ZK164015 (Tocris, CAS-No. 177583-70-9). The same protocols as in example 2 were used with the following deviations:

| | |
|---|---|
| Tamoxifen: | Cells were grown for 24h and then treated for 144h with 10⁻⁸ and 10⁻⁶ M tamoxifen. |
| MPP dihydrochloride: | Cells were grown for 24h and then treated for 144h with 10⁻⁸ and 10⁻⁶ M MPP dihydrochloride. |
| ZK164015: | Cells were grown for 24h and then treated for 48h with 10⁻⁹ and 10⁻⁷ M ZK164015. |

The result of this experiment is shown in figure 2. In analogy to fulvestrant, all three tested ERα antagonists trigger the formation of discontinuous adherence junctions between adjacent MCF7/vBOS cells throughout the epithelial monolayer within two to six days after treatment, as evidenced by E-Cadherin staining.

The MCF7/vBOS cell line is thus a suitable tool for testing a compound of interest for potential anti-estrogenic activity by exposing said cell line to the compound of interest and determining the adherens junction morphology of the cell, wherein the formation of discontinuous basolateral adherens junctions is indicative of anti-estrogenic activity.

### Example 4: Other MCF-7 cell lines do not show changes in basolateral adherens junctions morphology upon contact with anti-estrogenic compounds

Next, the inventors tried to reproduce the effects obtained with the cells as deposited under DSM ACC3321 with other conventional MCF-7 cells, namely MCF-7/BOS provided by Prof. Ana Soto (Tufts University School of Medicine, Boston, MA, USA) and the MCF-7/ACC115 clone obtained from the Leibniz-Institut DSMZ-Deutsche Sammlung von Mikroorganismen und Zellkulturen GmbH (Braunschweig, Germany).

Briefly, the MCF-7 cells were seeded and grown on glass coverslips in multi-well tissue culture plates (TPP) for 24h to reach near 100% confluence and then treated for at least 48h with 0.1% EtOH (solvent control) and the ERα antagonist fulvestrant (Sigma-Aldrich, CAS-No. 129453-61-8) at a concentration of 10⁻⁸ M. During experiments, medium was exchanged on a daily basis. All experiments were performed at 37°C with 5% CO₂ in reduced-serum medium (phenol red-free DMEM (Gibco), 5% FBS (Biochrom, S 0615, LOT1353 W), 1% glutamine, 100 µg/ml streptomycin / 100 U/ml penicillin (Biochrom)).

The result of this experiment is shown in figure 3. None of the conventional MCF-7 cells showed a comparable phenotype as the MCF7/vBOS cells deposited under DSM ACC3321. The reasons for this discrepancy are not known to the inventors.

### Example 5: Changes to adherens junctions morphology induced by fulvestrant can be prevented by 17-β-estradiol (E2)

In a next step, the inventors tried to elucidate whether the phenotype induced by exposure to anti-estrogenic compounds such as fulvestrant might be neutralized by stimulating estrogen signalling. For this purpose, the inventors used the estrogen 17-β-estradiol (E2) (Sigma-Aldrich, CAS-No. 50-28-2).

Briefly, MCF7/vBOS cells were grown on glass coverslips in multi-well tissue culture plates (TPP) for 24h and then treated for 48h with 0.1% EtOH (solvent control), the anti-estrogen fulvestrant in the fixed concentration of 10⁻⁸ M and different concentrations (10⁻¹⁰ M, 10⁻⁹ M, 10⁻⁸ M, 10⁻⁷ M) of the ERα agonist 17β-estradiol. The solvent control was used as a positive control and fulvestrant at a concentration of 10⁻⁸ M without 17β-estradiol addition was used as a negative control. Sample preparation and immunofluorescence microscopy was performed as described in example 2 above. After 48 hours the fraction of cells displaying discontinuous adherens junctions was determined by CellProfiler/CellProfer-analyst software (see example 7).

The result of this experiment is shown in figure 4 and 7. The analysis revealed that the fulvestrant-induced phenotype can be prevented by exposure to 17-β-estradiol (E2) at a minimal concentration of 10⁻⁹ M.

### Example 6: Changes to adherens junctions morphology induced by fulvestrant can also be prevented by estrogenic industrial chemicals

In view of the results obtained with MCF7/vBOS cells upon exposure to fulvestrant and 17-β-estradiol (E2) the inventors speculated that the phenotype inducible by anti-estrogens like fulvestrant could also be prevented with other estrogenic compounds. To test this hypothesis the inventors used the known estrogenic compounds bisphenol A (BPA) (Sigma-Aldrich, 239658) and nonylphenol (NP) (Sigma-Aldrich, 290858).

The same protocols as in example 2 were used with the following deviations:

| | |
|---|---|
| Bisphenol A: | Cells were grown for 24h and then treated for 48 h with 10⁻⁸ M fulvestrant and 10⁻⁵ or 10⁻⁴ M bisphenol A, respectively, diluted in DSMO. |
| Nonylphenol: | Cells were grown for 24h and then treated for 48 h with 10⁻⁸ M fulvestrant and 10⁻⁵ or 10⁻⁴ nonylphenol, respectively, diluted in ethanol. |

The result of this experiment is shown in figure 5 and 6. The estrogenic compounds bisphenol A and nonylphenol were both able to prevent the fulvestrant-induced phenotype at a minimal concentration of 10⁻⁵ M as at these concentrations the E-cadherin distribution was also similar to the ethanol treated solvent control.

Thus, the MCF7/vBOS cell line is also a tool suitable for testing a compound of interest for potential estrogenic activity, for example by exposing said cell line to fulvestrant or any other suitable anti-estrogen and to the compound of interest and determining the adherens junctions morphology of the cell, wherein a prevention of a discontinuous basolateral adherens junction phenotype is indicative of estrogenic activity.

### Example 7: Automated quantification of morphology changes at adherens junctions

To quantify the change in cell morphology upon fulvestrant treatment, the inventors implemented a software-based image analysis pipeline (software: CellProfiler; http://cellprofiler.org and CellProfiler Analyst, http://cellprofiler.org/cp-analyst/)) thereby greatly facilitating classification of cells into cells with continuous adherens junctions and discontinuous adherens junctions.

Briefly, cell culture, sample preparation and immunofluorescence microscopy was performed as described in example 2 (ERα signalling inhibition (fulvestrant titration)) and example 5 (ERα signalling restoration (fulvestrant at 10⁻⁸ M in combination with 17β-estradiol titration).

Subsequently, quantitative image analysis was performed using a pipeline that builds on the CellProfiler software (Carpenter et al., Genome Biol., 2006;7(10):R100.) for segmentation and extraction of cellular parameters, and on the CellProfiler Analyst software (Jones et al., BMC Bioinformatics, 2008 Nov 15;9:482) for parameter-based classification of cells. In the segmentation process, primary objects (nuclei) were identified from DAPI staining by global thresholding using Otsu's method. Next, primary objects served as seeding points for identification of secondary objects (cell membranes) at a defined distance. Finally, tertiary objects (cytoplasm) were defined by subtracting primary from secondary objects. The resulting object masks were then applied to extract cellular parameters including area, shape, distribution and variation of pixel intensities, and mean intensity from the corresponding greyscale images using the CellProfiler MeasureObjectSizeShape, MeasureObjectRadialDistribution, MeasureTexture, and MeasureObjectIntensity modules, and stored in a database file. This database file was then imported into the Classifier module of the CellProfiler Analyst software for supervised training with a subset of cells, and subsequent parameter-based automatic classification of all cells within the entire image dataset. For immunofluorescence images, supervised training based on cell membrane features (E-Cad channel) was conducted with two classes representing continuous adherens junctions (`Continuous AJs') and discontinuous adherens junctions ('Discontinuous AJs') (about 30 cells for each class). After the supervised training process had been completed, the entire image dataset from a specific experiment was evaluated and all identified cells were automatically classified into corresponding classes according to their individual parameters. The fraction of cells representing `Continuous AJs' and 'Discontinuous AJs' classes were finally graphically represented in stacked bar plots.

All graphical representations and statistical analyses of data were performed using the GraphPad Prism software (GraphPad Software, Inc.).

The result of this experiment is shown in figure 7. In accordance with the visual inspection of immunofluorescence images, upon treatment with different concentrations of fulvestrant, the fraction of cells with continuous adherens junctions decreased while the fraction of cells with discontinuous adherens junctions increased. In turn, fulvestrant-treatment in combination with different concentrations of 17-β-estradiol (E2) restores the fraction of cells with continuous adherens junctions.

Thus, morphological changes at adherens junctions can easily be monitored and evaluated even in an automated manner. This renders the methods of the present invention particularly suitable for high-throughput screening and automated image analysis.

### Example 8: Fulvestrant induced changes to adherens junction morphology can also be assessed by imaging of adherens junctions components other than E-cadherin

In a further set of experiments, the inventors assessed the impact of fulvestrant treatment on other components of the adherens junctions, namely β-Catenin, α-Catenin, p120-Catenin, and the cortical actin cytoskeleton. Briefly, the MCF-7/vBOS cells were seeded and grown on glass coverslips in multi-well tissue culture plates (TPP) for 24h to reach near 100% confluence and then treated for at least 48h with 0.1% EtOH (solvent control) and the ERα antagonist fulvestrant (Sigma-Aldrich, CAS-No. 129453-61-8) at a concentration of 10⁻⁸ M. During experiments, medium was exchanged on a daily basis. All experiments were performed at 37°C with 5% CO₂ in reduced-serum medium (phenol red-free DMEM (Gibco), 5% FBS (Biochrom, S 0615, LOT1353 W), 1% glutamine, 100 µg/ml streptomycin / 100 U/ml penicillin (Biochrom)). As a result, the change in adherens junctions morphology was also observable when staining any of β-Catenin, α-Catenin, p120-Catenin, or the cortical actin cytoskeleton.

### Example 9: Anti-estrogens elevate adherens junction stability

In a further set of experiments, the inventors tested whether the anti-estrogen-mediated reorganization of adherens junctions likewise increased adherens junction stability in MCF-7/vBOS cells. Therefore, EGTA-containing medium (8×10⁻³ M) was applied to both control cells and cells pre-treated for 48 hours with fulvestrant to disrupt homophilic E-Cadherin binding, and the fraction of rounded cells was monitored over the course of 120 minutes by live imaging. Quantification of rounded cells vs. non-rounded cells was done in analogous manner as described above in example 7 for quantification of continuous and discontinuous adherens junctions. Notably, cells pre-treated with fulvestrant showed a significantly reduced rate of cell rounding (Fig. 9) indicating that reorganized adherens junctions were stabilized and more resilient against disruption.

### Example 10: Anti-estrogens promote cell rigidity and reduce cell motility

To test whether anti-estrogens also impact on the biomechanical properties of MCF-7/vBOS breast cancer cells, the inventors performed atomic force microscopy (AFM) indentation measurements on confluent monolayers. For this purpose, MCF7/vBOS cells were grown and treated in 35 mm FluoroDish cell culture dishes (WPI) as described above. AFM indentation measurements were performed in CO₂ Independent Medium (Gibco) at 37°C using the NanoWizard 1 or 4 (JPK Instruments). Prior to measurements, tip-less Arrow-TL1silicone cantilevers (Nanoworld) were equipped with polystyrene beads of 5 µm in diameter (microParticles GmbH, Germany) using epoxy glue, and calibrated using built-in procedures of the SPM software (JPK Instruments). The cantilever was positioned above the confluent epithelial monolayer and lowered with a speed of 10 µm/s. In each experiment, force-distance curves (force setpoint 2.5 nN) from 3-4 different positions per cell were collected. Force-distance curves were transformed into force-versus-tip sample separation curves according to Radmacher (Methods Cell Biol 2002;68:67-90), and fitted with the Hertz/Sneddon model (27) for a spherical indenter using the JPK Data Processing software (JPK Instruments). A Poisson ratio of 0.5 was assumed for the calculation of the apparent elastic (Young's) modulus. To map the elastic modulus distribution, cells were probed with a spatial resolution of 1 µm using a MLCT cantilever (Bruker). Apparent elastic (Young's) moduli were determined using the Hertz model for a quadratic pyramid using the JPK Data Processing software (JPK Instruments).

Intriguingly, cell rigidity was significantly elevated throughout the cell surface and at junctional regions in fulvestrant-treated cells (Fig. 10A). Given that anti-estrogens increase adherens junction stability and cell rigidity, the inventors asked whether cell motility was likewise affected. Hence, the inventors recorded the motility of MCF-7/vBOS breast cancer cells over the course of 48 hours in a confluent monolayer after introducing a scratch nearby the imaged region. Notably, both the velocity of cells (Fig. 10B) and the length of cell trajectories (data not shown) were significantly reduced upon fulvestrant treatment. Together, the increased cell rigidity and reduced cell motility of MCF-7/vBOS breast cancer cells suggest that anti-estrogens can promote tissue stability and indicate a previously unknown mechanism mediating the metastasis-protective effect of anti-estrogens in endocrine therapy.

### Example 11: ERα signalling activity and adherens junction organization correlate in human breast cancer sections

The inventors tested whether the ERα signalling-dependent organization of adherens junctions can also be observed in breast cancer patients. Due to the poor availability of specimens from patients undergoing anti-estrogen-based endocrine therapy, the inventors utilized tissue microarrays (TMA) containing breast tissue sections from patients with diagnosed invasive ductal carcinoma (IDC). Using nuclear or cytoplasmic ERα localization as a surrogate for high or low ERα signalling activity, respectively, the inventors assessed the state of ERα signalling activity and the organization of adherens junctions by immunofluorescence microscopy.

Briefly, tissue microarrays containing 54 formalin-fixed paraffin-embedded breast cancer tissue sections (1.5 mm diameter) from 28 human patients with diagnosed invasive ductal carcinoma (IDC) (provided by Carsten Denkert; Institute for Pathology, Charité-Universitätsmedizin Berlin, Germany) were prepared for immunofluorescence microscopy according to Junghans et al. (Dev Dyn 2005;233:528-39). Tissue sections were subjected to immunofluorescence staining, imaging, and image analysis as described for the cell culture experiments.

As expected for IDC cells, all tumour samples expressed E-Cadherin as well as ERα, however, the organization of adherens junctions and the localization of ERα varied between patients. Notably, discontinuous adherens junction morphologies, that were reminiscent to the ones observed in MCF-7/vBOS breast cancer cells, were detectable in cells displaying low ERα signalling activity. Hence, the inventors applied the CellProfiler/CellProfiler-Analyst-based image analysis pipeline on all patient samples and quantified the fraction of IDC cells displaying continuous or discontinuous adherens junctions (Fig. 11, black bars), and nuclear or cytoplasmic ERα localization (Fig. 11, white bars). Intriguingly, the inventors found that the appearance of discontinuous adherens junctions correlated with primarily cytoplasmic ERα localization/ low ERα signalling activity, indicating that the organization of adherens junctions in vivo also seems to be dependent on ERα signalling activity.

## Claims

1. A cell as deposited under accession number DSM ACC3321 or a cell deriving from the cell as deposited under DSM ACC3321 and forming discontinuous basolateral adherens junctions in presence of an anti-estrogenic compound, wherein the cell deriving from the cell as deposited under DSM ACC3321 is a recombinant clone of said cell.

2. A method for testing a compound of interest for potential estrogenic activity and/or level of estrogenic activity, the method comprising the following steps:
a) growing a cell in presence of an anti-estrogenic compound and in in presence of the compound of interest, wherein growing the cell in presence of the anti-estrogenic compound may precede and/or occur in parallel to growing the cell in presence of the compound of interest, and
b) determining the adherens junctions morphology of the cell,
wherein the cell is a cell responding to the exposure to an anti-estrogenic compound by formation of discontinuous basolateral adherens junctions, and wherein prevention, reduction or reversion, respectively, of a discontinuous basolateral adherens junctions phenotype is indicative of estrogenic activity of the compound of interest.

3. The method of claim 2, wherein growing the cell in presence of the anti-estrogenic compound occurs in parallel to growing the cell in presence of the compound of interest.

4. The method of claim 2, wherein growing the cell in presence of the anti-estrogenic compound precedes growing the cell in presence of the compound of interest.

5. The method of any of claims 2 to 4, wherein the anti-estrogenic compound is selected from the group consisting of fulvestrant, tamoxifen, ZK 164015, and MPP dihydrochloride, in particular wherein the anti-estrogenic compound is fulvestrant.

6. A method for testing a compound of interest for potential anti-estrogenic activity and/or level of anti-estrogenic activity, the method comprising the following steps:
a) growing a cell in presence of the compound of interest, and
b) determining the adherens junctions morphology of the cell,
wherein the cell is a cell responding to the exposure to an anti-estrogenic compound by formation of discontinuous basolateral adherens junctions, and wherein a formation of discontinuous basolateral adherens junctions is indicative of anti-estrogenic activity.

7. The method of any of claims 2 to 6, wherein the cell is a cell according to claim 1.

8. A method for testing a cell for its potential to form discontinuous basolateral adherens junctions in presence of an anti-estrogenic compound, the method comprising the following steps:
a) growing the cell in presence of an anti-estrogenic compound, and
b) determining the adherens junctions morphology of the cell.

9. The method of claim 8, wherein the anti-estrogenic compound is selected from the group consisting of fulvestrant, tamoxifen, ZK 164015, and MPP dihydrochloride, in particular wherein the anti-estrogenic compound is fulvestrant.

10. The method of any of claims 2 to 9, wherein the cell is
i) a mammalian cell, preferably a human cell, and/or
ii) an adenocarcinoma cell, preferably a breast adenocarcinoma cell.

11. An ex vivo method for assessing the basolateral adherens junction morphology of cells in a tissue sample of a subject, the method comprising determining in the tissue sample of the subject the fraction of cells exhibiting discontinuous basolateral adherens junctions and/or determining in the tissue sample of the subject the fraction of cells exhibiting continuous basolateral adherens junctions.

12. An ex vivo method for assessing the metastatic potential of a tumour in a subject, the method comprising determining in a tissue sample of the tumour the fraction of cells exhibiting discontinuous basolateral adherens junctions and/or determining in the tissue sample of the tumour the fraction of cells exhibiting continuous basolateral adherens junctions, wherein a reduced fraction of cells exhibiting discontinuous basolateral adherens junctions compared to a control and/or an increased fraction of cells exhibiting continuous basolateral adherens junctions compared to a control indicates an elevated metastatic potential of the tumour.

13. The method of any of claims 2 to 12, wherein determining adherens junction morphology comprises the use of optical means.

14. The method of claim 13, wherein the optical means comprise fluorescence microscopy, in particular optical sectioning fluorescence microscopy.

15. The method of claim 13 or 14, wherein determining the adherens junctions morphology comprises detecting the distribution of E-cadherin at the adherens junctions of the cell.

16. The method of claim 15, wherein E-cadherin is detected by means of immunostaining or by use of labelled E-cadherin.

17. The method of claim 16, wherein E-cadherin is detected by means of fluorescently labelled E-cadherin.

## Patentansprüche

1. Unter der Hinterlegungsnummer DSM ACC3321 hinterlegte Zelle oder eine von der unter DSM ACC3321 hinterlegten Zelle abgeleitete Zelle, die in Gegenwart einer antiöstrogenen Verbindung diskontinuierliche basolaterale Adhäsionsverbindungen bildet, wobei die von der unter DSM ACC3321 hinterlegten Zelle abgeleitete Zelle ein rekombinanter Klon dieser Zelle ist.

2. Verfahren zum Testen einer interessierenden Verbindung auf potenzielle östrogene Aktivität und/oder den Grad der östrogenen Aktivität, wobei das Verfahren die folgenden Schritte umfasst:
a) Züchten einer Zelle in Gegenwart einer antiöstrogenen Verbindung und in Gegenwart der interessierenden Verbindung, wobei das Züchten der Zelle in Gegenwart der antiöstrogenen Verbindung dem Züchten der Zelle in Gegenwart der interessierenden Verbindung vorausgehen und/oder parallel erfolgen kann, und
b) Bestimmen der Morphologie der adhärenten Verbindungen der Zelle,
wobei es sich bei der Zelle um eine Zelle handelt, die auf die Einwirkung einer antiöstrogenen Verbindung durch die Bildung diskontinuierlicher basolateraler adhärenter Verbindungen reagiert, und wobei die jeweilige Verhinderung, Verringerung oder Umkehrung des Phänotyps diskontinuierlicher basolateraler adhärenten Verbindungen ein Hinweis auf die östrogene Aktivität der interessierenden Verbindung ist.

3. Verfahren nach Anspruch 2, wobei das Züchten der Zelle in Gegenwart der antiöstrogenen Verbindung parallel zum Züchten der Zelle in Gegenwart der interessierenden Verbindung erfolgt.

4. Verfahren nach Anspruch 2, wobei das Züchten der Zelle in Gegenwart der antiöstrogenen Verbindung dem Züchten der Zelle in Gegenwart der interessierenden Verbindung vorausgeht.

5. Verfahren nach einem der Ansprüche 2 bis 4, wobei die antiöstrogene Verbindung aus der Gruppe bestehend aus Fulvestrant, Tamoxifen, ZK 164015 und MPP-Dihydrochlorid ausgewählt ist, insbesondere wobei die antiöstrogene Verbindung Fulvestrant ist.

6. Verfahren zum Testen einer interessierenden Verbindung auf mögliche antiöstrogene Aktivität und/oder den Grad der antiöstrogenen Aktivität, wobei das Verfahren die folgenden Schritte umfasst:
a) Züchten einer Zelle in Gegenwart der interessierenden Verbindung und
b) Bestimmen der Morphologie der adhärenten Verbindungen der Zelle,
wobei es sich bei der Zelle um eine Zelle handelt, die auf die Einwirkung einer antiöstrogenen Verbindung durch die Bildung diskontinuierlicher basolateraler adhärenten Verbindungen reagiert, und wobei die Bildung diskontinuierlicher basolateraler adhärenter Verbindungen auf eine antiöstrogene Aktivität hinweist.

7. Verfahren nach einem der Ansprüche 2 bis 6, wobei die Zelle eine Zelle nach Anspruch 1 ist

8. Verfahren zum Testen einer Zelle auf ihr Potenzial zur Bildung diskontinuierlicher basolateraler Adhäsionsverbindungen in Gegenwart einer antiöstrogenen Verbindung, wobei das Verfahren die folgenden Schritte umfasst:
a) Züchten der Zelle in Gegenwart einer antiöstrogenen Verbindung und
b) Bestimmen der Morphologie der adhärenten Verbindungen der Zelle,

9. Verfahren nach Anspruch 8, wobei die antiöstrogene Verbindung aus der Gruppe bestehend aus Fulvestrant, Tamoxifen, ZK 164015 und MPP-Dihydrochlorid ausgewählt ist, insbesondere wobei die antiöstrogene Verbindung Fulvestrant ist.

10. Verfahren nach einem der Ansprüche 2 bis 9, wobei die Zelle
i) eine Säugetierzelle, vorzugsweise eine menschliche Zelle, und/oder
ii) eine Adenokarzinomzelle, vorzugsweise eine Brustadenokarzinomzelle.
ist.

11. Ex-vivo-Verfahren zur Beurteilung der Morphologie der basolateralen adheränten Verbindungen von Zellen in einer Gewebeprobe eines Subjekts, wobei das Verfahren das Bestimmen des Anteils von Zellen, die diskontinuierliche basolaterale adheränte-Verbindungen aufweisen, in der Gewebeprobe des Subjekts und/oder das Bestimmen des Anteil von Zellen, die kontinuierliche basolaterale adhärente Verbindungen aufweisen, in der Gewebeprobe des Subjekts umfasst.

12. Ex-vivo-Verfahren zur Beurteilung des Metastasierungspotentials eines Tumors in einem Subjekt, wobei das Verfahren das Bestimmen des Anteils von Zellen, die diskontinuierliche basolaterale adheränte Verbindungen aufweisen, in der Gewebeprobe des Subjekts und/oder das Bestimmen des Anteil von Zellen, die kontinuierliche basolaterale adhärente Verbindungen aufweisen, in der Gewebeprobe des Subjekts umfasst, wobei ein verringerter Anteil von Zellen, die diskontinuierliche basolaterale adhärente Verbindungen im Vergleich zu einer Kontrolle aufweisen, und/oder ein erhöhter Anteil von Zellen, die kontinuierliche basolaterale adhärente Verbindungen aufweisen, im Vergleich zu einer Kontrolle auf ein erhöhtes Metastasierungspotenzial des Tumors hinweist.

13. Verfahren nach einem der Ansprüche 2 bis 12, wobei die Bestimmung der Morphologie der adhärenten Verbindung die Verwendung optischer Mittel umfasst.

14. Verfahren nach Anspruch 13, wobei die optischen Mittel Fluoreszenzmikroskopie umfassen, insbesondere optische Schnittfluoreszenzmikroskopie.

15. Verfahren nach Anspruch 13 oder 14, wobei die Bestimmung der Morphologie der adhärenten Verbindungen das Erfassen der Verteilung von E-Cadherin an den adhärenten Verbindungen der Zelle umfasst.

16. Verfahren nach Anspruch 15, wobei E-Cadherin mittels Immunfärbung oder durch Verwendung von markiertem E-Cadherin nachgewiesen wird.

17. Verfahren nach Anspruch 16, wobei E-Cadherin mittels fluoreszenzmarkiertem E-Cadherin nachgewiesen wird.

## Revendications

1. Cellule telle que déposée sous le numéro d'enregistrement DSM ACC3321 ou cellule dérivée de la cellule telle que déposée sous le numéro d'enregistrement DSM ACC3321 et formant des jonctions adhérentes basolatérales discontinues en présence d'un composé anti-œstrogénique, ladite cellule dérivée de la cellule telle que déposée sous le numéro d'enregistrement DSM ACC3321 étant un clone recombinant de ladite cellule.

2. Méthode permettant de tester un composé d'intérêt pour une activité oestrogénique potentielle et/ou un taux d'activité oestrogénique, ladite méthode comprenant les étapes suivantes :
a) la culture d'une cellule en présence d'un composé anti-œstrogénique et en présence du composé d'intérêt, la culture de la cellule en présence du composé antiœstrogénique pouvant précéder et/ou se produire en parallèle à la culture de la cellule en présence du composé d'intérêt, et
b) la détermination de la morphologie des jonctions adhérentes de la cellule,
la cellule étant une cellule répondant à l'exposition à un composé antiœstrogénique par la formation de jonctions adhérentes basolatérales discontinues, et la prévention, la réduction ou le renversement, respectivement, d'un phénotype de jonctions adhérentes basolatérales discontinues étant indicatif de l'activité oestrogénique du composé d'intérêt.

3. Méthode selon la revendication 2, dans laquelle la culture de la cellule en présence du composé anti-œstrogénique s'effectue en parallèle à la culture de la cellule en présence du composé d'intérêt.

4. Méthode selon la revendication 2, dans laquelle la culture de la cellule en présence du composé anti-œstrogénique précède la culture de la cellule en présence du composé d'intérêt.

5. Méthode selon l'une quelconque des revendications 2 à 4, dans laquelle le composé anti-œstrogénique est choisi dans le groupe se composant du fulvestrant, du tamoxifène, du ZK 164015 et du dichlorhydrate de MPP, en particulier dans laquelle le composé anti-œstrogénique est le fulvestrant.

6. Méthode permettant de tester un composé d'intérêt pour une activité potentielle anti-œstrogénique et/ou un taux d'activité anti-œstrogénique, la méthode comprenant les étapes suivantes :
a) la culture d'une cellule en présence du composé d'intérêt, et
b) la détermination de la morphologie des jonctions adhérentes de la cellule,
la cellule étant une cellule répondant à l'exposition à un composé anti-œstrogénique par la formation de jonctions adhérentes basolatérales discontinues, et une formation de jonctions adhérentes basolatérales discontinues étant indicative d'une activité anti-œstrogénique.

7. Méthode selon l'une quelconque des revendications 2 à 6, dans laquelle la cellule est une cellule selon la revendication 1.

8. Méthode permettant de tester une cellule pour son potentiel à former des jonctions adhérentes basolatérales discontinues en présence d'un composé antiœstrogénique, ladite méthode comprenant les étapes suivantes :
a) la culture de la cellule en présence d'un composé anti-œstrogénique, et
b) la détermination de la morphologie des jonctions adhérentes de la cellule.

9. Méthode selon la revendication 8, dans laquelle le composé antiœstrogénique est choisi dans le groupe se composant du fulvestrant, du tamoxifène, du ZK 164015 et du dichlorhydrate de MPP, en particulier dans laquelle le composé antiœstrogénique est le fulvestrant.

10. Méthode selon l'une quelconque des revendications 2 à 9, dans laquelle la cellule est
i) une cellule de mammifère, de préférence une cellule humaine, et/ou
ii) une cellule d'adénocarcinome, de préférence une cellule d'adénocarcinome du sein.

11. Méthode ex vivo permettant d'évaluer la morphologie des jonctions adhérentes basolatérales des cellules dans un échantillon de tissu d'un sujet, ladite méthode comprenant la détermination, dans l'échantillon de tissu du sujet, de la fraction des cellules présentant des jonctions adhérentes basolatérales discontinues et/ou la détermination, dans l'échantillon de tissu du sujet, de la fraction des cellules présentant des jonctions adhérentes basolatérales continues.

12. Méthode ex vivo permettant d'évaluer le potentiel métastatique d'une tumeur chez un sujet, ladite méthode comprenant la détermination, dans un échantillon de tissu du sujet, de la fraction de cellules présentant des jonctions adhérentes basolatérales discontinues et/ou la détermination, dans l'échantillon de tissu du sujet, de la fraction de cellules présentant des jonctions adhérentes basolatérales continues, une fraction réduite de cellules présentant des jonctions adhérentes basolatérales discontinues par rapport à un témoin et/ou une fraction accrue de cellules présentant des jonctions adhérentes basolatérales continues par rapport à un témoin indiquant un potentiel métastatique élevé de la tumeur.

13. Méthode selon l'une quelconque des revendications 2 à 12, dans laquelle la détermination de la morphologie des jonctions adhérentes comprend l'utilisation de moyens optiques.

14. Méthode selon la revendication 13, dans laquelle les moyens optiques comprennent la microscopie de fluorescence, en particulier la microscopie de fluorescence à sectionnement optique.

15. Méthode selon la revendication 13 ou 14, dans laquelle la détermination de la morphologie des jonctions adhérentes comprend la détection de la distribution de la E-cadhérine au niveau des jonctions adhérentes de la cellule.

16. Méthode selon la revendication 15, dans laquelle la E-cadhérine est détectée au moyen d'une immunocoloration ou par l'utilisation de E-cadhérine marquée.

17. Méthode selon la revendication 16, dans laquelle la E-cadhérine est détectée au moyen de la E-cadhérine marquée par fluorescence.
